# EUROPEAN PATENT APPLICATION

(11) **EP 4 480 501 A1**
(43) Date of publication of application: **25.12.2024**
(21) Application number: 23180747.0
(22) Date of filing: 21.06.2023
(51) Int. Cl.: A61K 51/10, C07K 16/28

(54) **SIRP NANOBODIES**

(71) Applicant: NMI NATURWISSENSCHAFTLICHES UND MEDIZINISCHES INSTITUT AN DER UNIVERSITÄT TÜBINGEN, 72770 Reutlingen (DE); Eberhard-Karls-Universität Tübingen, 72074 Tübingen (DE)
(72) Inventor: Rothbauer, Ulrich, 72072 Tübingen (DE); Tränkle, Björn, 72800 Eningen (DE); Kaiser, Philipp, 72076 Tübingen (DE); Wagner, Teresa, 72116 Mössingen (DE); Sonanini, Dominik, 72072 Tübingen (DE); Kneiling, Manfred, 85298 Scheyern (DE); Pichler, Bernd, 85298 Fernhag (DE)
(74) Representative: Witte, Weller & Partner Patentanwälte mbB

(57) **Abstract**

The present invention relates to nanobodies, which bind to human SIRPα as well as to the use of the nanobodies in diagnostic or therapeutic applications.

## Description

The present invention relates to novel single-domain-antibodies (sdAb), which are also known as nanobodies, which bind to a member of the human SIRP family, wherein the nanobody binds at least to human SIRPα, as well as to the use of such nanobodies, in particular for prophylactic, therapeutic, diagnostic or cell-targeting purposes.

The invention also relates to nucleic acids encoding such nanobodies, to compositions comprising such nanobodies or polypeptides, and to their uses, in particular for prophylactic, therapeutic, diagnostic or cell-targeting purposes.

### BACKGROUND

Proteins of the SIRP family are immune checkpoint proteins and are paired receptors, with similar extracellular domains but different transmembrane and cytoplasmic regions. The SIRP family comprises inhibitory (SIRP alpha or SIRPα or CD172a), activating (SRIP beta or SIRPβ), and non-signaling (SIRP gamma or SIRPγ) members; amongst them, SIRPaV1 and SIRPaV2 are the main SIRPα variants in human.

The best studied SIRP family member is SIRP alpha. SIRP alpha expression is restricted to myeloid cells while its binding partner CD47 is expressed on T cells. Reagents blocking the SIRP-alpha/CD47 interaction could activate macrophages or dendritic cells to stimulate phagocytosis and to eliminate tumor cells. Preclinical studies have suggested that SIRP alpha or CD47 blockade may have synergistic antitumor effects in combination with other immune checkpoint inhibitors that target the adaptive immune system. SIRP gamma also binds to CD47, albeit with lower affinity than SIRP alpha. While a SIRP beta ligand remains unknown, SIRP beta has been shown to associate with the adaptor protein DAP12 via a basic amino acid motif in its cytoplasmic region leading to activation phagocytosis by macrophages.

The Signal-regulatory protein α (SiRPα), as mentioned above also known as CD172a or SHPS-1, is an innate immune checkpoint inhibitor predominantly expressed on myeloid cells like monocytes, macrophages, dendritic cells and neutrophils as well as neurons and stem cells. Structurally SIRPα consists of three Ig-like domains (domain d1, domain d2, domain d3), a transmembrane domain and a cytoplasmic domain containing two Immunoreceptor Tyrosine-Based Inhibitory Motifs (ITIMs) with four tyrosine residues that are potential sites for phosphorylation.

SIRPα recognizes CD47 which is ubiquitously expressed on most human cell types. CD47 overexpression was observed in various hematological and solid cancer types including non-Hodgkin lymphoma, acute myeloid leukemia, glioblastoma, ovarian, breast, colon, bladder, hepatocellular, and prostate cancer. The extracellular domain d1 of SIRPα binds to CD47 and transmits intracellular signals through its cytoplasmic domain. The four cytoplasmic ITIMs of SIRPα become phosphorylated after binding of the ligand CD47. Upon binding CD47, SIRPα initiates a signaling cascade that results in the inhibition of phagocytosis and prevention of subsequent autoimmune responses. The interaction of SIRPα and CD47, thus, mediates a "don't eat me" signal, by means of which tumor cells, often overexpressing CD47, avoid phagocytosis by tumor-associated macrophages.

Currently, there are different strategies for manipulating the SIRPα/CD47 interaction, which include, e.g., blocking anti-CD47 monoclonal antibodies or blocking anti-SIRPα monoclonal antibodies. However, even though some of the recent approaches show that an antibody-dependent cellular phagocytosis (ADCP) of tumor cells could be triggered, some cases have also shown that using blocking anti-CD47 antibodies bears the risk of causing a decrease of CD47-expressing red blood cells. Also, other antibodies have not proven to be as effective as intended, while causing undesirable side effects. Thus, there still remains the need for an improved therapeutical approach in treating diseases, such as cancer, chronic, infectious and and inflammatory diseases.

### SUMMARY

The present invention provides a nanobody (or single-domain-antibody), which binds to a member of the human SIRP family, wherein the nanobody binds at least to human SIRPα and wherein the nanobody does not have cross-reactivity with murine SIRPα, the nanobody comprising:
a) the amino acid sequences (i) EFRLDEYA (SEQ ID NO: 1) as CDR1, (ii) ISRSGSNT (SEQ ID NO: 2) as CDR2 and (iii) AADLTSHYLSCSSYTDYNS (SEQ ID NO: 3) as CDR3; or
b) the amino acid sequences (i) GFSSSYYA (SEQ ID NO: 4) as CDR1, (ii) ITRSGDTT (SEQ ID NO: 5) as CDR2 and (iii) ALWAWAGSGRLRCTASEYGH (SEQ ID NO: 6) as CDR3; or
c) the amino acid sequences (i) GFTLDYYS (SEQ ID NO: 7) as CDR1, (ii) ITASGSST (SEQ ID NO: 8) as CDR2 and (iii) AAEPCTWAGILaAPTSOFGS (SEQ ID NO: 9) as CDR3; or
d) the amino acid sequences (i) GFTLDYYA (SEQ ID NO: 10) as CDR1, (ii) ISSDGGGNT (SEQ ID NO: 11) as CDR2 and (iii) AAGPTYYSGSYCRDPHSDYDY (SEQ ID NO: 12) as CDR3; or
e) the amino acid sequences (i) GETLDYYA (SEQ ID NO: 13) as CDR1, (ii) ISSRGDNT (SEQ ID NO: 14) as CDR2 and (iii) VAIKDSWQNYYCSVGWVEYDV (SEQ ID NO: 15) as CDR3; or
f) the amino acid sequences (i) GFTFSSYD (SEQ ID NO: 16) as CDR1, (ii) IKSGGGRT (SEQ ID NO: 17) as CDR2 and (iii) GSLDNSGAYTG (SEQ ID NO: 18) as CDR3; or
g) the amino acid sequences (i) GFSFRDSA (SEQ ID NO: 19) as CDR1, (ii) IYSDGST (SEQ ID NO: 20) as CDR2 and (iii) APKGVVADEGDY (SEQ ID NO: 21) as CDR3; or
h) the amino acid sequences (i) GRTFRAYA (SEQ ID NO: 32) as CDR1, (ii) IRWIGGTP (SEQ ID NO: 33) as CDR2 and (iii) AAEETQKGSGLGHDGRHYDY (SEQ ID NO: 34) as CDR3; or
i) amino acid sequences that have at least 85% sequence homology with the amino acid sequences as defined in a), b), c), d), e), f), g), or h),
or a functionally conservative variant of the nanobody as defined in any of a), b), c), d), e), f), g), or h) comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 10, SEQ ID No. 11, and SEQ ID No. 12; or SEQ ID No. 13, SEQ ID No. 14, and SEQ ID No. 15; or SEQ ID No. 16, SEQ ID No. 17, and SEQ ID No. 18; SEQ ID No. 19, SEQ ID No. 20, and SEQ ID No. 21; or SEQ ID No. 32, SEQ ID No. 33, and SEQ ID No. 34.

The novel single domain antibodies (or "nanobodies") of the invention specifically recognize at least human SIRPα in its native state on various human SIRPα⁺ cells. With the novel nanobodies disclosed herein, it is possible to selectively identify or target SIRPα⁺ cells, such as monocytes and macrophages, which makes them a valuable tool in diagnosis, prophylaxis, and therapy, alone or in combination/association with additional substances/molecules or methods.

A single domain antibody (sdAb) is an antibody fragment consisting of a single monomeric variable antibody domain, which is able to selectively bind to a specific antigen. As single domain antibodies, the nanobodies of the invention represent an excellent alternative to conventional antibodies (IgGs): Antibodies from camelids, such as llamas, include a unique subset of immunoglobulins consisting of heavy chain homodimers devoid of light chains. Their variable region (V_{H}H) is the smallest antigen-binding fragment found in the antibody world, and as a single polypeptide chain it is especially suitable for protein engineering. Single domain antibodies, or "nanobodies", are the recombinant minimal-sized, intact antigen-binding domains derived from the V_{H}H region of these heavy-chain antibodies. Unlike monoclonal antibodies, they can be readily produced in large amounts in simple bacterial expression systems. Moreover, nanobodies are usually extremely stable, can bind antigens with affinities in the nanomolar range, and are smaller in size (approximately 15 kDa) and thereby easier to manipulate genetically as compared with antibody fragments such as single chain variable fragments (ScFvs) comprising the variable domains of heavy and light chains of a conventional IgG.

Also, due to the small size and compact folding nanobodies show a high chemical stability, solubility, and fast tissue penetration. Additionally, nanobodies, in particular the ones according to the invention, can be easily converted into multivalent formats, such as biparatopic, bivalent, trivalent, tetravalent or other multi-specific constructs, e.g., addressing different epitopes on the same antigen, which the nanobodies presented herein can also be used for according to the invention. Also, nanobodies have comparable antigen specificities and affinities and, due to their high homology with human antibody (VH) fragments, show only very low immunogenicity.

Accordingly, the nanobodies of the present invention are not antibodies that occur naturally in the human body, but represent synthetically, i.e., biotechnologically generated and engineered antibody fragments.

Throughout the present disclosure, and unless indicated otherwise, the expressions "SIRPα nanobody" and "human SIRPα nanobody" or "hSIRPα nanobody" are interchangeably used and all three expressions designate the same, i.e., a nanobody directed against at least human SIRPα, or, in other words, a nanobody binding to at least human SiRPα. According to the invention, the nanobody does not have any cross-reactivity towards murine SiRPα.

In this context, the expression "nanobody directed against at least human SIRPα" or "nanobody binding to at least human SIRPα" is meant to comprise a nanobody that binds to only human SIRPα, and a nanobody that binds - in addition to binding to human SIRPα - to one or more other member(s) of the SIRP family. Known members of the SIRP family are, e.g.: hSiRPα; two isoforms of hSIRPα, i.e., hSiRPαV1 and hSiRPαV2; hSIRPβ1; hSIRPγ.

Thus, in preferred embodiments of the invention, the nanobody can bind to only hSIRPα; or to hSIRPα and to hSiRPαV1 and/or to hSiRPαV2; or to hSIRPα and to hSIRPβ1; or to hSIRPα and to hSIRPβ1 and to hSiRPαV1 and/or to hSiRPαV2; or to hSIRPα and to hSIRPγ; or to hSIRPα and to hSIRPγ and to hSiRPαV1 and/or to hSIRPaV2 and/or to hSIRPβ1.

Accordingly, throughout the present disclosure, a SIRPα⁺ cell is meant to designate any cell expressing at least SIRPα as member of the SIRP family.

According to an embodiment of the present invention, the nanobody or nanobodies of the invention binds to domain d1 or to domain d2 or to domain d3 of the extracellular region human SiRPα.

Human SIRPα, being a transmembrane protein, has an extracellular portion or region, which consists of three immunoglobulin superfamily (IgSF) domains d1, d2 and d3. The N-terminal (IgSF V-set) domain, mediating binding to CD47, is designated as d1, and the two other domains, being membrane-proximal (d2 and d3), are constant (IgSF C1-set) domains. The structure and the domains of SIRPα have been characterized and are known in the art (see, e.g., Hatherley et al., "Structure of Signal-regulatory Protein α", J Biol Chem (2009) 284(39):26613-26619), and it is referred to the structure and domain characterization of human SIRPα as acknowledged in the scientific publications of the prior art.

Further, an exemplary exhaustive disclosure for hSIRPα, its names, taxonomy, domains, etc., and also for its amino acid sequences is given in the UniProt database under the ID no. P78324 ("SHPS1_human"). As exemplarily disclosed in this database, the amino acid sequence spanning amino acid no. 21 to 137 of hSIRPα is assigned to domain d1, the amino acid sequence spanning amino acid no. 148 to 247 of hSIRPα is assigned to domain d2, and the amino acid sequence spanning amino acid no. 254 to 348 of hSIRPα is assigned to domain d3, with hSIRPα having a total length of 504 amino acids.

According to one aspect of the invention, the nanobody of the invention is specifically binding to d1 or d2 or d3 of human SIRPα, the specific binding of which can be tested or shown, e.g., by means of using cells expressing the individual domains of human SIRPα and by determining the domain specificity of the nanobody by immunofluorescence staining and/or hydrogen deuterium exchange coupled mass spectrometry analysis.

In addition, the epitope diversity can be determined via epitope binning analysis and/or hydrogen deuterium exchange coupled mass spectrometry analysis.

Also, according to an embodiment of the present invention, the nanobody is an hSIRPα-CD47-interaction-blocking nanobody; a myeloid- and/or T-cell-depleting nanobody; or an inert, functionally non-modulating hSIRPα nanobody.

Presently, and as generally understood, a "hSIRPα-CD47-interaction blocking SIRPα nanobody" is a nanobody that does block, *in vivo* and *in vitro,* the binding of CD47 to hSIRPα by binding to hSIRPα, thus inhibiting the initiation of the signaling cascade, and, thus, inducing phagocytosis. Without the blocking, CD47 can bind to hSIRPα thereby inhibiting phagocytosis.

In an embodiment of the present invention, the hSIRPα-CD47-interaction blocking SIRPα nanobody binds to domain d1 of hSIRPα. Preferably, the hSIRPα-CD47-interaction blocking SIRPα nanobody is a nanobody comprising/having the CDR amino acid sequences of the above e): (i) GETLDYYA (SEQ ID NO: 13) as CDR1, (ii) ISSRGDNT (SEQ ID NO: 14) as CDR2 and (iii) VAIKDSWQNYYCSVGWVEYDV (SEQ ID NO: 15) as CDR3; or f) (i) GFTFSSYD (SEQ ID NO: 16) as CDR1, (ii) IKSGGGRT (SEQ ID NO: 17) as CDR2 and (iii) GSLDNSGAYTG (SEQ ID NO: 18) as CDR3; or g): the amino acid sequences (i) GFSFRDSA (SEQ ID NO: 19) as CDR1, (ii) IYSDGST (SEQ ID NO: 20) as CDR2 and (iii) APKGWADEGDY (SEQ ID NO: 21) as CDR3e), f) or g); or amino acid sequences that have at least 85% sequence homology with the amino acid sequences as defined in e), f), g), or a functionally conservative variant of the nanobody as defined in any of e), f), or g), comprising a conservative substitution of one or two amino acids in one, two or three of the sequences, respectively, SEQ ID No. 13, SEQ ID No. 14, and SEQ ID No. 15; or SEQ ID No. 16, SEQ ID No. 17, and SEQ ID No. 18; or SEQ ID No. 19, SEQ ID No. 20, and SEQ ID No. 21.

In an embodiment of the present invention, the nanobody is a myeloid- and/or T-cell-depleting nanobody, which is preferably selected from a SIRPα nanobody comprising/having the CDR amino acid sequences of the above a), b), c), d), e), f), g), h) or i).

Presently, and as generally understood, a "myeloid- and/or T cell-depleting nanobody" is a nanobody that can remove or reduce these cells in a sample (*in vitro*) or patient (*in vivo*)*.* Herein, and as generally understood in hematopoiesis, myeloid or my-elogenous cells are blood cells that arise from a progenitor cell for granulocytes (neutrophils, eosinophils, basophils), monocytes (macrophages, dendritic cells), erythrocytes, or platelets, the aforementioned representing exemplary embodiments of a myeloid cell.

The *in vivo* depletion of these cells can alter the immune system and its responses. Thus, according to the present invention, the nanobodies of the invention can be used for treatment/therapeutic purposes of diseased patients.

E.g., T cell depletion can be used to reduce the risk of graft-versus-host disease (GVHD) in transplantations, or, generally, to treat autoimmune-related diseases related to the above cells. An "autoimmune-related disease" is a disease caused by excessive activation and/or proliferation of various immune cells, such as effector T cells or cytotoxic T cells and inflammatory cells, and may include, but is not limited to, as mentioned, autoimmune diseases, graft-versus-host diseases, organ transplant rejection diseases, asthma, atopy, or acute or chronic inflammatory diseases.

According to the present invention, the "autoimmune disease" may be at least one selected from the group consisting of, but not limited to, rheumatoid arthritis, type 1 diabetes, systemic scleroderma, systemic lupus erythematosus, atopic dermatitis, psoriasis, alopecia areata, asthma, crohn's disease, behcet's disease, chronic thyroiditis, multiple sclerosis, polymyositis, ankylosing spondylitis, fibromyositis, and polyarteritis.

Additional diseases or medical conditions that can be treated according to the invention are, e.g., macrophage activation syndrome and hemophagocytic lympho-histiocytosis, immune mediated inflammatory diseases (e.g., ulcerative colitis), bacterial and viral infections (.e.g., hyperinflammatory syndrome), lung fibrosis, acute respiratory distress syndrome, cancer therapy (e.g., in lung cancer, sarcoma; see also further below), preparative immunosuppression for adoptive cell transfer (e.g. chimeric antigen receptor T cell therapies), glomerulonephritis, leukemia (in particular acute myelomonocytic leukemia), neurodegenerative diseases (e.g., Alzheimer disease), vascular diseases (e.g. atherosclerosis), ischemic heart disease, myocardial infarction and ischemic stroke.

Presently, and as generally understood, an "inert nanobody"/"inert hSIRPα nanobody" is a nanobody, that does not block the hSIRPα-CD47-interaction upon binding to hSIRPα. Such nanobodies are useful for diagnostic purposes, e.g., as radiolabeled tracers or fluorescent labeled probes for non-invasive *in vivo* imaging.

In an embodiment of the present invention, the inert hSIRPα nanobody can bind to domain d1, d2, or d3 of hSIRPα.

In an embodiment of the present invention, the inert hSIRPα nanobody is a nanobody comprising/having the CDR amino acid sequences of the above a): (i) EFRLDEYA (SEQ ID NO: 1) as CDR1, (ii) ISRSGSNT (SEQ ID NO: 2) as CDR2 and (iii) AADLTSHYLSCSSYTDYNS (SEQ ID NO: 3) as CDR3; or b): (i) GFSSSYYA (SEQ ID NO: 4) as CDR1, (ii) ITRSGDTT (SEQ ID NO: 5) as CDR2 and (iii) ALWAWAGSGRLRCTASEYGH (SEQ ID NO: 6) as CDR3; or c): (i) GFTLDYYS (SEQ ID NO: 7) as CDR1, (ii) ITASGSST (SEQ ID NO: 8) as CDR2 and (iii) AAEPCTVVAGILQAPTSDFGS (SEQ ID NO: 9) as CDR3; or d): (i) GFTLDYYA (SEQ ID NO: 10) as CDR1, (ii) ISSDGGGNT (SEQ ID NO: 11) as CDR2 and (iii) AAGPTYYSGSYCRDPHSDYDY (SEQ ID NO: 12) as CDR3; or h) (i) GRTFRAYA (SEQ ID NO: 32) as CDR1, (ii) IRWIGGTP (SEQ ID NO: 33) as CDR2 and (iii) AAEETQKGSGLGHDGRHYDY (SEQ ID NO: 34); or amino acid sequences that have at least 85% sequence homology with the amino acid sequences as defined in a), b), c), d), or h) or a functionally conservative variant of the nanobody as defined in any of a), b), c), d) or h), comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 10, SEQ ID No. 11, SEQ ID No. 12, SEQ ID NO: 32, SEQ ID NO: 33 and SEQ ID NO: 34.

Generally, the amino acid sequence and structure of nanobodies can be considered to include, but are not limited to, four framework regions or "FRs", which are interrupted by three complementarity determining regions or "CDRs". The total number of amino acid residues in a nanobody may be preferably in the range of 110-130.

Thus, according to an embodiment of the present invention, the nanobody comprises four framework regions (FR1 to FR4) and three complementarity determining regions (CDR1 to CDR3), the three complementarity determining regions consisting of
(i) one of the amino acid sequences a), b), c), d), e), f), g), or h), or of
(ii) an amino acid sequence that has at least 85% sequence homology with one of the amino acid sequences a), b), c), d), e), f), g), h), or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 10, SEQ ID No. 11, and SEQ ID No. 12; or SEQ ID No. 13, SEQ ID No. 14, and SEQ ID No. 15; or SEQ ID No. 16, SEQ ID No. 17, and SEQ ID No. 18; or SEQ ID No. 19, SEQ ID No. 20, and SEQ ID No. 21; SEQ ID No. 32, SEQ ID No. 33, and SEQ ID No. 34.
wherein preferably, the nanobody comprises or consists of an amino acid sequence selected from the group consisting of the amino acid sequences SEQ ID NO. 22, SEQ ID NO. 23, SEQ ID NO. 24, SEQ ID No. 25, SEQ ID No. 26, SEQ ID No. 27, SEQ ID No. 28, SEQ ID NO. 29, SEQ ID No. 30, SEQ ID No. 31, SEQ ID NO. 35; or wherein the nanobody comprises or consists of an amino acid sequence that has at least 85% sequence homology with one of the amino acid sequences SEQ ID NO. 22, SEQ ID NO. 23, SEQ ID No. 24 or SEQ ID No. 25, SEQ ID No. 26, SEQ ID No. 27, SEQ ID No. 28; SEQ ID NO. 35.

In a preferred embodiment of the invention, the nanobody has a sequence homology in the four framework regions (FR1 to FR4) of at least 60% , and a sequence homology in the three complementarity determining regions (CDR1 to CDR3) of at least 85%.

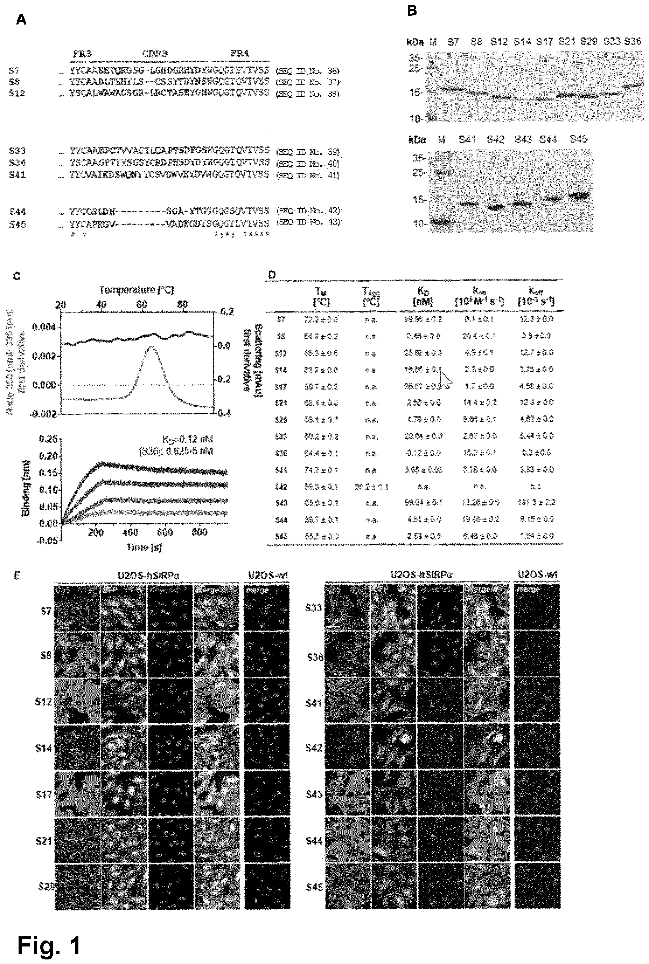

The amino acid sequences SEQ ID nos. 1 to 21, and 32, 33, and 34, represent CDRs specific examples for the nanobody/nanobodies of the invention, and the SEQ ID nos. 22 to 28, and 35 represent the sequences of the respective full-length nanobody examples, representing specific embodiments of the present invention. SEQ ID No. 29 represents an exemplary embodiment of a nanobody having a sequence homology of about 95% with SEQ ID No. 22; SEQ ID NO. 30 represents an exemplary embodiment of a nanobody having a sequence homology of about 96% with SEQ ID No. 24; SEQ ID No. 31 exemplary embodiment of a nanobody having a sequence homology of about 96,8% with SEQ ID No. 25. SEQ ID Nos. 36 to 43 designate the truncated sequences of SEQ ID Nos. 22 to 28 and 35, respectively, shown in Fig. 1A.

In a preferred embodiment, the nanobody of the invention has a level of affinity to SRIPα, expressed in the dissociation constant (K_{D}), of between including 0,1 nM to including 26 nM. As generally known, the higher the level of affinity of a nanobody/protein to its ligand, the lower the dissociation constant (K_{D}) of the nanobody/ligand-complex. The nanobody of the invention, thus, has a very high level of affinity.

In a preferred embodiment, the nanobody of the invention has a level of affinity to SRIPα, expressed in the dissociation constant (K_{D}), of between including 0.1 nM, preferably 2 nM, to including 6 nM wherein, preferably, the nanobody is a hSIRPα-CD47-interaction blocking SIRPα nanobody.

The dissociation constant (K_{D}) can be determined, e.g., by affinity measurements against recombinant hSIRPα by biolayer interferometry (BLI), using, e.g., the Octet BLI system (Sartorius, Göttingen, Germany), e.g., the Octet RED96e system, as also outlined in the experimental section.

With the exemplarily disclosed nanobodies, the inventors could show that not only an effective binding to primary human myeloid cells could be achieved, but also a functionally blocking of the hSIRPα-CD47-interaction could be effected. Further, within the context of the present invention, it was shown that the nanobodies of the invention, an even augmented phagocytosis of tumor cells in the presence of an additional anticancer agent, which could be specifically shown for the nanobodies of the invention having SEQ ID nos. 26, 27, 28.

According to a preferred embodiment of the invention, the nanobody is a humanized variant of a nanobody of the invention.

According to an embodiment of the present invention, the hSIRPα nanobodies are modified, such, that the binding affinity of the modified nanobodies to hSIRPα is substantially retained as compared to the unmodified hSIRPα nanobody, while the hSIRPα-CD47-interaction remains unblocked/non-modulated. Accordingly, with such a modification of the nanobodies of the invention, inert hSIRPα nanobodies can be provided, which can be used for, e.g., non-invasive *in vivo* imaging of hSIRPα expression.

The nanobodies of the present invention, as well as the nucleic acids coding for them, i.e., the nucleic acids of the present invention, encompass polypeptides/proteins and nucleic acids having the sequences specified, or sequences substantially identical or similar thereto, e.g., sequences at least 85%, 86%, 87%, 88%, 89% 90%, 91%, 92%, 93%, 94%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences containing a common structural domain having at least about 85%, 86%, 87%, 88%, 89%, 90%, 91%, 92%, 93%, 94%, 95%, 96%, 97%, 98% or 99% identity to a reference sequence. In other embodiments, the amino acid sequence can contain one or more amino acid insertions, deletions, or substitutions (e.g., conservative substitutions) to arrive at a percentage identity of at least about 85%, 86%, 87%, 88%, 89%, 90%, 91 %, 92%, 93%, 94%, 95%, 96%, 97%, 98% or 99% identity to a reference sequence. In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity or encode a common structural polypeptide domain or a common functional polypeptide activity. For example, nucleotide sequences having at least about 80%, 85%, 90%, 91%, 92%, 93%, 94%, 95%, 96%, 97%, 98% or 99% identity to a reference sequence.

Accordingly, a "functionally conservative variant" of the nanobody of the invention as used herein means a nanobody or fragment in which one or more amino acid residues have been modified without altering desired properties, such as antigen affinity and/or antigen specificity. Such variants include, but are not limited to, certain amino acids substituted with amino acids having similar properties. E.g., a "conservative amino acid substitution" is one in which the amino acid residue is replaced with an amino acid residue having a similar side chain. Families of amino acid residues having similar side chains have been defined in the art. Such conservative substituents are preferably substituted by another amino acid residue of the following groups (a) to (e): (a) a small aliphatic, nonpolar or weakly polar residue (Ala, Ser Thr, Pro, Gly), (b) polar, negatively charged residues and their (un-charged) amides (Asp, Asn, Glu, Gln), (c) polar, positively charged residues (His, Arg, Lys) ), (D) large aliphatic, nonpolar residues (Met, Leu, Ile, Val, Cys), and (e) aromatic residues (Phe, Tyr, Trp).

Preferably, an amino acid substitution is performed by means of which the primary amino acid, via which a chemical coupling can take place, is exchanged.

Particularly preferred conservative substitutions are: Ala to Gly or Ser, Arg to Lys, Asn to Gln or His, Asp to Glu, Cys to Ser, Gln to Asn, Glu to Asp, Gly to Ala or Pro, His To Asn or Gin, lie to Leu or Val, Leu to lie or Val, Lys to Arg or Gln or Glu, Met to Leu or Tyr or Ile, Phe to Met or Leu or Tyr, Ser to Thr, Thr to Ser, Trp To Tyr, Tyr to Trp, and / or Phe to Val or lie or Leu, wherein a particularly preferred substitution is Lys to Arg.

According to preferred embodiment, the nanobody of the invention is modified, such, that in the amino acid sequences of the nanobody lysine residues are exchanged with arginine.

In a preferred embodiment, the modified nanobody comprises or consists of an amino acid sequence selected from the group consisting of the amino acid sequences SEQ ID NO. 29, SEQ ID NO. 30 or SEQ ID NO. 31, corresponding to the modified version (lysine (K) -> arginine (R)) of SEQ ID nos. 22, 24 and 25, respectively.

In an embodiment of the invention, the nanobody is associated with at least one of a detectable label, and/or a therapeutically or pharmacologically active agent.

With the modified nanobodies, the applicability of the nanobodies of the invention as radiolabeled "tracer-like" means to visualize and monitor the distribution of hSIRPα cells or generally tumors by non-invasive *in vivo* PET could be shown: The nanobodies of the invention, which have been labeled with a radionuclide, and tested *in vitro,* as well as *in vivo* regarding their ability to accumulate in tumor tissue and organs with increased densities of myeloid cells such as the spleen and bone marrow. With the nanobodies of the invention, a strong accumulation in the tumor organs with increased densities of myeloid cells of mice could be shown.

In an embodiment of the present invention, the detectable label is selected from detectable moieties for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

The term "associated with" when used in connection with the nanobody of the invention refers to any modification of the nanobody with another moiety that is/can be used as a diagnostic moiety/agent, or as a therapeutically or pharmacologically active moiety or agent. With the modification, the agent or moiety (the nanobody gets associated with) is indirectly or directly bound to the nanobody or incorporated into the nanobody.

For example, such modification may comprise the introduction (e.g. by conjugation, biologically or chemically linking, covalent binding or in any other suitable manner) of one or more functional groups or agents or moieties, which can be a detectable label, a viral particle, and/or a therapeutically or pharmacologically active agent, and/or that, e.g., alters/increases the half-life, the solubility and/or the absorption of the nanobody, that reduce the immunogenicity and/or the toxicity of the nanobody, that eliminate or attenuate any undesirable side effects of the nanobody, and/or that confer other advantageous properties to and/or reduce the undesired properties of the nanobody; or any combination of two or more of the foregoing. Examples of such functional groups and of techniques for introducing them will be clear to the skilled person and can generally comprise all functional groups and techniques mentioned in the general background art cited hereinabove as well as the functional groups and techniques known *per se* for the modification of pharmaceutical proteins, and in particular for the modification of antibodies or antibody fragments. Such functional groups may for example be linked directly (for example covalently) to a nanobody used in the invention, or optionally via a suitable linker or spacer, as will again be clear to the skilled person.

As used herein the terms "label", "marker" and variants thereof when used in the context of a nanobody/construct refer to a detectable compound, composition or molecule that is linked, associated, or conjugated directly or indirectly to the nanobody or construct disclosed herein, to facilitate detection of the compound. Non-limiting examples of labels as known in the art include fluorescent tags, enzymatic linkages, and radioactive isotopes. In one example, a "labeled nanobody" refers to the direct or indirect conjugation of the detectable compound, composition, or molecule to the nanobody. Additionally, or alternatively, the detectable compound, composition or molecule can be incorporated into the nanobody structure by means other than direct or indirect conjugation. An exemplary method of such incorporation is the replacement of an amino acid residue of the nanobody with a modified amino acid residue such that it becomes detectable (e.g., by radiolabeling). The label may be directly detectable or may be detectable only after contact with further compounds compositions or molecules. In one, non-limiting example, the label may be the incorporation of a radiolabeled amino acid, which is directly detectable according to methods known in the art. In additional or alternative non-limiting examples, the label may be the attachment of biotinyl moieties to the nanobody, which are detectable following contact with marked avidin (for example, streptavidin containing a fluorescent marker or enzymatic activity that can be detected by optical or colorimetric methods as known in the art). Various methods of labeling proteins are known in the art and may be used.

In an embodiment of the invention, in particular where the nanobody of the invention is used in diagnostic imaging methods, the nanobody is associated with a functional entity via which a detectable entity can be attached to the nanobody. E.g., the nanobody of the invention is combined with a chelator, and via the chelator, e.g., a radionuclide is attached to the nanobody. The chelator can be any "bifunctional chelating agent" having the capability to chelate (radio-)metal ions and to covalently bind the nanobody/nanobodies of the invention. Exemplary bifunctional chelating agents include, but are not limited to, DTPA, NOTA, benzyl-NOTA, alkyl or aryl derivatives of NOTA, NODA, NODA-GA, C-NETA, succinyl-C-NETA and bis-t-butyl-NODA. As mentioned, via the chelator, e.g., a radionuclide can be attached to/associated with the nanobody of the invention, in particular with the radionuclides as discussed below.

According to an embodiment of the invention, examples for detectable labels for polypeptides/proteins include, but are not limited to, the following: radioisotopes or radionuclides (such as ³⁵S, ¹¹C, ¹³N, ¹⁵O, ¹⁸F, ¹⁹F, ⁶⁴Cu, ⁹⁹TC, ¹³¹I, ³H, ¹⁴C, ¹⁵N, ⁸⁹Zr, ⁹⁰Y, ⁹⁹Tc, ¹¹¹In and ¹²⁵I), fluorescent labels (such as fluorescein isothiocyanate (FITC), rhodamine, lanthanide phosphors), enzymatic labels (such as horseradish peroxidase, beta-galactosidase, luciferase, alkaline phosphatase), chemiluminescent markers, biotinyl groups, predetermined polypeptide epitopes recognized by a secondary reporter (such as a leucine zipper pair sequences, binding sites for secondary antibodies, metal binding domains, epitope tags), or magnetic agents (such as gadolinium chelates). In some embodiments, labels are attached by spacer arms of various lengths to reduce potential steric hindrance for the binding of the nanobody or the construct disclosed herein.

To "label" a nanobody of the invention, any method known in the field can be applied. Generally, to label the nanobody of the invention for, e.g., positron emission tomography (PET), PET tracers are coupled to a chelator (e.g., NODAGA) or click-reagent via a reactive unit (e.g., primary amino group, azide group in combination with DBCO, C-terminal cysteine, His6-tag) located within the nanobody, via which the radionuclide is then introduced.

According to an embodiment, the nanobody of the invention may be labelled, e.g., via a chelator as discussed above, with an radio-isotope chosen from the group consisting of Actinium-225, Astatine-211 , Bismuth-212, Bismuth-213, Caesium-137, Chromium-51 , Cobalt-60, Dysprosium -165, Erbium-169, Fermium-255, Gold-198, Holium-166, lodine-125, lodine-131 , Iridium-192, Iron-59, Lead-212, Lutetium-177, Mo-lydenum-99, Palladium-103, Phosphorus-32, Potassium-42, Rhenium-186, Rhenium-188, Samarium-153, Technitium-99m, Radium-223 , Ruthenium-106, Sodium-24, Strontium-89, Terbium-149, Thorium-227, Xenon-133, Ytterbium-169, Ytterbium-177, Yttrium-90.

According to one aspect of the invention, the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

With the nanobody of the invention modified with a detectable label or "a contrast agent", different approaches for imaging cells can be performed. E.g., noninvasive imaging approaches offer a significant benefit compared to current diagnostic standard, since, currently, e.g., suitable rodent models for *in vivo* application of hSIRPα nanobodies are not available.

According to another embodiment, as mentioned above, the nanobody of the invention can be associated with a viral particle, and the thus generated construct can be efficiently used for targeting hSIRPα⁺ myeloid cells, and can be used, e.g., for gene therapy, preferably for gene therapy of disease-related myeloid hSIRPα⁺ cells.

A gene transfer agent allows clinicians to introduce a therapeutic relevant nucleic acid sequence (i.e., DNA or RNA) of interest directly into a patient (*in vivo* gene therapy) or into cells isolated from a patient or a donor (*ex vivo* gene therapy). Therapeutic transgenes (encoding polypeptides, including nanobodies), produced by transduced cells after gene therapy can be maintained at a relatively constant level in a subject, as compared to a protein that is administered directly. Expression can be transient (on the order of hours to weeks) or sustained (weeks to months or longer), depending upon the specific construct used and the target tissue or cell type. These transgenes can be introduced as an integrating or non-integrating linear construct, a circular plasmid, a viral vector. Moreover, these transgenes can be introduced by virus-like particles (VLP) or chemical nanoparticle carriers (e.g. lipid nanoparticles; LNP). Also, with the nanobody of the invention, genetically engineered T cells expressing chimeric antigen receptors (CAR) can be generated, which then can be used in therapy of, e.g., cancer.

Also, as presently understood, a "therapeutically or pharmacologically active agent" may comprise any therapeutically active agent that effects a therapeutic, i.e., beneficial treatment effect in a patient in need of a therapeutic treatment. Non-limiting examples of agents include, for example, a cytokine inhibitor, a growth factor inhibitor, an immunosuppressant, an anti-inflammatory agent, a metabolic inhibitor, an enzyme inhibitor, a cytotoxic agent, a radiolabel-agent, and a cytostatic agent.

The invention also concerns a nucleic acid comprising or consisting of a nucleic acid sequence coding for the nanobody of the invention, optionally linked to another nucleic sequence.

The present invention also relates to a nucleic acid encoding an amino acid sequence comprising framework region 1, CDR1, framework region 2, CDR2, framework region 3, CDR3 and framework region 4, in this order, of the nanobody as described herein, as well as a polypeptide comprising at least one nanobody as described herein.

In some of the aspects described herein, a nucleic acid sequence encoding a nanobody of the invention, or any module thereof, is operably linked to a vector. In general, as used herein, the term "vector" refers to any genetic element, such as a plasmid, phage, transposon, cosmid, chromosome, virus, virion, etc., that is capable of replication when associated with the proper control elements and that can transfer gene sequences to cells. Thus, the term includes cloning and expression vehicles, as well as viral vectors. By "recombinant vector" is meant a vector that includes a heterologous nucleic acid sequence, or "transgene", that is capable of expression *in vivo.* Vectors useful for the delivery of a sequence encoding a nanobody of the invention or components thereof can include one or more regulatory elements (e.g., promoter, enhancer, etc.) sufficient for expression of the nanobody or component thereof in the desired target cell or tissue.

In an embodiment, the nanobody of the invention, or the nucleic acid of the invention, is/are used, as already addressed above, in diagnostic or prognostic methods, preferably imaging methods, in or for a patient, or used as medicament, preferably for blocking the interaction of hSIRPα with CD47, or for target cell depletion, wherein the target cell preferably is a myeloid cell or T-cell, with the myeloid cell preferably representing a blood cell that arises from a progenitor cell for granulocytes (neutrophils, eosinophils, basophils), monocytes (macrophages, dendritic cells), erythrocytes, or platelets, the aforementioned representing exemplary embodiments of a myeloid cell..

By "diagnostic method" or "diagnosis", is meant here a method giving the possibility of determining whether an individual suffers from a pathology or certain status, e.g., treatment status, or disease status, in particular in view of a cancer/tumor pathology or cancer/tumor disease of a patient.

By "prognostic method" or "prognosis", is meant here a method giving the possibility of determining whether an individual risks developing a pathology, in particular in view of a cancer/tumor pathology or cancer/tumor disease of a patient.

The patient, as discussed in and throughout the present invention, is preferably a mammal, in particular a mammal selected from a human or an animal mammal, the latter preferably being selected from non-human primates, rodents, in particular rats, mice.

Preferably, in an embodiment of the invention, the nanobody of is used as contrast agent in non-invasive medical imaging *in vivo,* preferably for identifying the presence of SIRPα+ cells, further preferably for identifying the presence and/or infiltration of a SIRPα+ myeloid cell population within a tumor microenvironment in a patient or a sample of a patient.

By "use as a medicament" or drug, the general use of the nanobody as medicament or drug is meant, i.e., for treating a disease or medical condition or pathology. The use can comprise a treatment method comprising the administration of a therapeutically effective amount of the nanobody or the construct of the invention to a patient in need thereof. According to an embodiment, the patient is a mammal, preferably a human.

The diseases or conditions that may be therapeutically treated and/or monitored using the nanobody of the invention are selected from cancer, viral and bacterial infections, autoimmune diseases, colitis, allogenic stem cells transplantation, organ transplant rejection, acquired immunodeficiency disease, and also include the ones listed above. The "autoimmune disease" may be at least one selected from the group consisting of, but not limited to, rheumatoid arthritis, type 1 diabetes, systemic scleroderma, systemic lupus erythematosus, atopic dermatitis, psoriasis, alopecia areata, asthma, crohn's disease, behcet's disease, chronic thyroiditis, multiple sclerosis, polymyositis, ankylosing spondylitis, fibromyositis, and polyarteritis.

In an embodiment, the nanobody of the invention is used in the treatment of a solid tumor, cancer, leukemia, a lymphoma, an inflammatory disease, a neurological disorder, or a chronic infection.

By "solid tumor(s)" or "tumor(s)" are meant primary tumors and/or metastases (wherever located) such as but not limited to gliomas, pancreatic tumors; lung cancer, e.g. small cell lung cancer, breast cancer; epidermoid carcinomas; neuroendo-crine tumors; gynecological and urological cancer, e.g. cervical, uterine, ovarian, prostate, renal-cell carcinomas, testicular germ cell tumors or cancer; pancreas cancer (pancreatic adenocarcinoma); glioblastomas; head and/or neck cancer; CNS (central nervous system) cancer; bones tumors; solid pediatric tumors; hematological malignancies; AIDS-related cancer; soft-tissue sarcomas, and skin cancer, including melanoma and Kaposi's sarcoma.

As used herein, the term "cancer cell" refers to a cell that divides and reproduces abnormally and limitlessly with uncontrolled growth and which can break away and travel to other parts of the body and set up another site, referred to as metastasis.

Non-limiting examples of cancers are: Examples of cancers or neoplastic conditions include, but are not limited to, a fibrosarcoma, myosarcoma, liposar-coma, chondrosarcoma, osteogenic sarcoma, gastric cancer, esophageal cancer, rectal cancer, pancreatic cancer, ovarian cancer, prostate cancer, uterine cancer, cancer of the head and neck, skin cancer, brain cancer, squamous cell carcinoma, medullary carcinoma, bronchogenic carcinoma, renal cell carcinoma, hepatoma, bile duct carcinoma, cervical cancer, testicular cancer, small cell lung carcinoma, non-small cell lung carcinoma, bladder carcinoma, epithelial carcinoma, glioma, astrocytoma, medulloblastoma, melanoma, neuroblastoma, retinoblastoma, leukemia, lymphoma, or Kaposi sarcoma.

The therapeutic use of the nanobody also includes using the nanobody for target cell depletion, wherein the target cell preferably is a myeloid cell or T cell. The myeloid cell, as mentioned above, is preferably selected from granulocytes (e.g., neutrophils, eosinophils, basophils), monocytes (e.g., macrophages, dendritic cells), erythrocytes, or platelets.

The *in vivo* depletion of these cells can alter the immune system and its responses and can be used/induced for treatment purposes of diseased patients. E.g., T cell depletion can be used to reduce the risk of graft-versus-host disease (GVHD) in transplantations, or, generally, to treat autoimmune-related diseases related to the above cells. An "autoimmune-related disease" is a disease caused by excessive activation and/or proliferation of various immune cells, such as effector T cells or cytotoxic T cells and inflammatory cells, and may include, but is not limited to, as mentioned, autoimmune diseases, graft-versus-host diseases, organ transplant rejection diseases, asthma, atopy, or acute or chronic inflammatory diseases.

According to the present invention, the "autoimmune disease" may be at least one selected from the group consisting of, but not limited to, rheumatoid arthritis, type 1 diabetes, systemic scleroderma, systemic lupus erythematosus, atopic dermatitis, psoriasis, alopecia areata, asthma, crohn's disease, behcet's disease, chronic thyroiditis, multiple sclerosis, polymyositis, ankylosing spondylitis, fibromyositis, and polyarteritis.

Additional diseases or medical conditions that can be treated according to the invention are, e.g., macrophage activation syndrome and hemophagocytic lympho-histiocytosis, immune mediated inflammatory diseases (e.g., ulcerative colitis), bacterial and viral infections (.e.g., hyperinflammatory syndrome), lung fibrosis, acute respiratory distress syndrome, cancer therapy (e.g., in lung cancer, sarcoma; see also further below), preparative immunosuppression for adoptive cell transfer (e.g. chimeric antigen receptor T cell therapies), glomerulonephritis, leukemia (in particular acute myelomonocytic leukemia), neurodegenerative diseases (e.g., Alzheimer disease), vascular diseases (e.g. atherosclerosis), ischemic heart disease, myocardial infarction and ischemic stroke.

According to an aspect of the invention, the nanobody is used in combination with at least one therapeutically or pharmacologically active agent, wherein preferably the therapeutically or pharmacologically active agent is selected from the group consisting of an anticancer or antitumor agent, wherein further preferably the antitumor agent is a therapeutic antibody.

The combined use of the nanobody of the invention, together with at least one therapeutically or pharmacologically active agent can lead to a synergistic effect.

In the context of this invention, "in combination with" shall mean that the nanobodies of the invention are applied together with one or more other pharmaceutically active compounds to a patient in need thereof in a regimen wherein the patient may profit from the beneficial effect of such a combination. In particular, both treatments are applied to the patient in temporal proximity. In a preferred embodiment, both treatments are applied to the patient within four weeks (28 days). More preferably, both treatments are applied within two weeks (14 days), more preferred within one week (7 days). In a preferred embodiment, the two treatments are applied within two or three days. In another preferred embodiment, the two treatments are applied at the same day, i.e., within 24 hours. In another embodiment, the two treatments are applied within four hours, or two hours, or within one hour. In another embodiment, the two treatments are applied in parallel, i.e., at the same time, or the two administrations are overlapping in time.

In an embodiment of the present invention, the nanobody/nanobodies of the invention is/are used in combination with one or more therapeutic antibodies or thera-peutic antibody fragments, such as but not limited to a combination treatment with bevaci-zumab, ramucirumab, pertuzumab, trastuzumab, cetuximab, nimotuzumab, panitumumab, necitumumab, dinutuximab,rituximab, ibritumomab, ofatumumab, obinutuzumab, alemtuzumab, daratumumab, gemtuzumab, elotuzumab, brentuximab, inotuzumab ozo-gamicin, and blinatumomab.

In an embodiment, the nanobody of the invention is used in combination with at least one therapeutically or pharmacologically active agent which is selected from the group consisting of one or more of methotrexate, cisplatin, carboplatin, paclitaxel, docetaxel, fluorouracil, vinorelbine, bleomycin, ifosfamide, mesna, leucovorin, mitoxan-trone, pirarubicin, daunorubicin, cytarabine, thioguanine, etoposide, harringtonine, gemcitabine, cetuximab, and epirubicin, doxorubicin, 5-fluorouracil.

The nanobody of the invention may for example be administered via a parenteral route in a suitable form. When the parenteral route is contemplated, the nanobody may be in the form of injectable solutes and suspensions packaged in ampoules or flasks. The forms for parenteral administration are conventionally obtained by mixing the nanobody with buffers, stabilizers, preservatives, solubilizing agents, isotonic agents, and suspension agents. According to known techniques, these mixtures are then sterilized and then packaged in the form of intravenous injections. The administered amount of nanobody or the construct naturally depends on the administration method, on the size and/or on the weight of the patient, and on the nature of the therapeutically active agent which may be associated therewith.

The present invention also concerns a multiparatopic, biparatopic, bivalent or trivalent construct comprising at least one nanobody of the invention or a combination of several nanobodies of the invention.

A "multiparatopic construct" as used herein and as generally understood refers to a polypeptide construct that binds to two or more different epitopes on one antigen, e.g., two (= biparatopic) or three (= triparatopic). Accordingly, the present invention also comprises constructs comprising the binding sequences of at least two of the nanobodies of the invention.

A "multivalent construct", on the other hand, is a construct that has at least two antigen binding sites/binding units. In some embodiments, a multivalent construct is a bivalent construct, trivalent construct, or a tetravalent construct.

Within the present invention, "at least one nanobody" includes "only one nanobody" of the invention, but also more than one nanobodies of the invention, e.g., 2, 3, 4, 5, 6 or 7 different nanobodies of the invention.

The present invention also concerns a method for determining and/or imaging and/or monitoring the distribution, presence, absence, and/or amount of SIRPα⁺ cells in a biological sample or tissue, the method comprising: contacting the biological sample or tissue with at least one nanobody of the invention and determining and/or imaging and/or monitoring the distribution, presence, absence, and/or amount of SIRPα⁺ cells in the biological sample or tissue. Preferably, the biological sample and tissue is from/in a human patient. The tissue is preferably an organ of a human patient.

In particular, in a preferred embodiment of the invention, the method comprises the steps of
a) contacting said biological sample or tissue with at least one of the nanobodies of the present invention, wherein said nanobody is associated with a detectable label as outlined above,
b) measuring, via the detectable label, imaging signals from said nanobody that has bound to SIRPα⁺ cells in said biological sample or tissue, and
c) imaging, determining and/or monitoring the presence, absence, and/or amount and/or activity of SIRPα⁺ cells, in said biological sample or tissue via the imaging signals,
thereby determining and/or monitoring the distribution of SIRPα⁺ cells in the biological sample or tissue.

The biological sample can be any sample or a portion of a larger biological element. Preferably, the sample is a substance of biological origin. Examples of biological samples include, but are not limited thereto, portions of organs or tissues such as the kidney, the liver, heart, the lung, etc., the arteries, the veins, etc., the blood, e.g. whole blood, and its compounds such as the plasma, serum, the platelets, the sub-populations of blood cells etc., as well as saliva, sputum, or any sample retrieved from the mouth and pharynx region, also e.g. by using a mouth wash or rinse containing saliva or sputum samples. The biological sample, preferably, is from a mammal, preferably a human. Also, preferably, the biological sample can be a cell culture comprising cells derived from a mammal, preferably a human, such as primary cells or (established) cell lines, with a "primary cell culture" being the culture of cells directly isolated from a tissue of interest and retaining the morphological and functional characteristics of their tissue of origin. Whereas cell lines are cells that have been continually passaged over a long period of time and have acquired homogenous genotypic and phenotypic characteristics; they can be finite or continuous.

The tissue can be any tissue of a mammal, preferably a human, i.e., the method can be performed on living mammals, e.g., a human, so that non-invasive *in vivo* imaging can be performed. By applying this method, e.g., tumor growth or size can be determined and monitored.

In an embodiment of the method of the invention, the nanobody is detectably labeled, preferably radiolabeled, and in the determining and/or imaging and/or monitoring step the presence, or absence, or distribution, or amount of the label is detected.

Accordingly, the present invention also relates to the use of at least one of the nanobodies of the present invention, or of at least one construct of the present invention, as a diagnostic agent, preferably wherein the diagnostic agent is used in a diagnosis which is selected from diagnostic classification of a disease-associated status of a patient, susceptibility to a medical therapy, such as an immunotherapy, of a patient.

In another embodiment of the method of the invention, the at least one nanobody of the present invention, or at least one construct of the present invention, is used to assess and/or monitor SIRPα+ cells in a biological sample of the patient, wherein the nanobody is used alone, or in combination with, or sequentially relative to at least one other, i.e., second, third, fourth, fifth, sixth, seventh, eight, nineth, tenth, etc., cell-specific marker. The at least one other cell-specific marker can be any marker of interest and suitable for detecting a cell/cells of interest expressing SIRPα and the second cell-specific marker.

In an embodiment of the combined use, the second cell-specific marker is a compound, e.g. an antibody/nanobody or fragment thereof, binding to a T-cell marker wherein the T cell-specific marker is selected from at least one of CD2, CD3, CD7, CD8, CD27, CD28, CD127, HLA-DR (MHC II), CD38, CD69, CCR4, CCR5, CCR6, CCR7, CTLA-4, LAG3, TIM-3, OX40, ICOS, CXCR4, CXCR1, CXCR2, PD-1, PD-L1, PD-L2, CD40L (synonym CD154), CD122, CD137, GITR, CD25, CD278, SIGLEC-7, SIGLEC-9, BTLA (synonym: CD272), TIGIT, VISTA, B7-H4 (synonym: VTCN1), CD276 (synonym: B7-H3), A2AR, CEACAM1, LAIR-3, HVEM, CD160, CD200, CD200R, CD206 (MMR), CD163, CD11a, CD11b, CD11c, CD14, CD86, CD34, CD45, CD80, IL-1R, IL-4 R alpha, TLR1, TLR2, TLR4, CD31, CD68, CD15, Fc gamma RI (CD64), Fc gamma RII (CD32), Fc gamma RIII (CD16), EMR1, galectin-3, M-CSF R/CD115, Siglec-3/CD33, CD36, INF gamma R, CD209, CD150.

The present invention also concerns a pharmaceutical composition comprising at least one of the nanobodies of the invention, and/or at least one construct of the invention, the pharmaceutical composition optionally further comprising: (i) a pharmaceutically acceptable carrier or excipient, and/or (ii) at least one therapeutically or pharmacologically active agent, wherein preferably the therapeutically or pharmacologically active agent is selected from the group consisting of an anticancer or antitumor agent, wherein further preferably the anti-tumor agent is a therapeutic antibody.

"Pharmaceutically acceptable" means those which are useful in preparing a pharmaceutical composition that is generally safe, non-toxic and neither biologically nor otherwise undesirable, and that is acceptable for human pharmaceutical use when the carriers are included.

Thus, the term "pharmaceutically" or "pharmaceutically acceptable" refers to molecular entities and compositions which do not produce secondary, allergic, or other unfortunate reactions when they are administered to a mammal, in particular a human. Accordingly, a "pharmaceutically acceptable carrier" is understood to mean any excipient, additive, or vehicle that is typically used in the field of the treatment of diseases, and which simplifies or enables the administration of the product according to the invention to a living being, preferably a human, and/or improves its stability and/or activity. The pharmaceutical composition can also incorporate binding agents, diluting agents or lubricants. The selection of a pharmaceutical carrier or other additives can be made on the basis of the intended administration route and standard pharmaceutical practice. As pharmaceutical acceptable carrier use can be made of/comprise one or more of solvents, extenders, or other liquid binding media such as dispersing or suspending agents, surfactant, isotonic agents, spreaders or emulsifiers, preservatives, encapsulating agents, solid binding media, lyoprotectants, bulking agents, stabilizer, depending upon what is best suited for the respective dose regime and is likewise compatible with the compound according to the invention. An overview of such additional ingredients can be found in, for example, Rowe (Ed.) et al.: Handbook of Pharmaceutical Excipients, 7th edition, 2012, Pharmaceutical Press.

"Pharmaceutically acceptable salt" includes, but not limited to acid addition salt formed from inorganic acid, such as hydrochloric acid, hydrobromic acid, sulfuric acid, nitric acid, phosphoric acid and the like; or acid addition salt formed from organic acid, such as acetic acid, trifluoroacetic acid, propionic acid, caproic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methyl sulfonic acid, ethyl sulfonic acid, 1,2-ethanedisulfonic acid, 2-hydroxyethanesulphonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid, stearic acid and the like.

The pharmaceutical composition can, e.g., comprise an aqueous buffer at a physiologically acceptable pH (e.g., pH 7 to 8.5), a polymer-based nanoparticle vehicle, a liposome, and the like. The pharmaceutical compositions can be delivered in any suitable dosage form, such as a liquid, gel, solid, cream, or paste dosage form.

Any administration method, known to one skilled in the art, may be used for administrating the nanobody or the pharmaceutical composition according to the invention to the patient. In particular, the nanobody/the pharmaceutical composition may be for example administered orally, by inhalation or via a parenteral route, in particular by injection, e.g., subcutaneous, intravascular, intramuscular or intraperitoneal. When the parenteral route is selected, the nanobody/pharmaceutical composition may be in the form of injectable solutions and suspensions, packaged in ampoules or flasks. The parenteral administration forms are conventionally obtained by mixing the nanobody according to the invention with buffers, stabilizers, preservatives, solubilizing agents, isotonic agents and suspending agents. According to known techniques, these mixtures may be sterilized or packaged as intravenous injections. One skilled in the art may for example use a buffer based on phosphate salts as buffers.

A "lyoprotectant" is a molecule which, when combined with a protein of interest, significantly prevents, or reduces chemical and/or physical instability of the protein upon lyophilization and subsequent storage. Exemplary lyoprotectants include sugars such as sucrose, sorbitol, or trehalose; an amino acid such as monosodium glutamate or histidine; a methylamine such as betaine; a lyotropic salt such as magnesium sulfate; a polyol such as trihydric or higher sugar alcohols, e.g., glycerin, erythritol, glycerol, arabitol, xylitol. A "stabilizer" refers to a molecule which, when combined with a protein of interest (e.g., the nanobody/single domain antibody molecule) substantially prevents or reduces chemical and/or physical instability of the protein of interest in lyophilized, reconstituted, liquid or storage form. Exemplary stabilizers include sucrose, sorbitol, glycine, inositol, sodium chloride, methionine, arginine, and arginine hydrochloride. A "diluent" of interest herein is one which is pharmaceutically acceptable (safe and non-toxic for administration to a human) and is useful for the preparation of a reconstituted formulation. Exemplary diluents include sterile water, bacteriostatic water for injection (BWFI), a pH buffered solution (e.g., phosphate-buffered saline), sterile saline solution, Ringer's solution or dextrose solution. A "preservative" is a compound which can be added to the diluent to essentially reduce bacterial action in the reconstituted formulation, thus facilitating the production of a multi-use reconstituted formulation, for example. Examples of potential preservatives include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl and benzyl alcohol, alkyl para-bens such as methyl or propyl paraben, catechol, resorcinol, cyclohexanol, 3-pentanol, and m-cresol. The most preferred preservative herein is benzyl alcohol. A "bulking agent" is a compound which adds mass to the lyophilized mixture and contributes to the physical structure of the lyophilized cake (e.g., facilitates the production of an essentially uniform lyophilized cake which maintains an open pore structure). Exemplary bulking agents include mannitol, glycine, polyethylene glycol and sorbitol.

The administered amount of nanobodies/the pharmaceutical composition naturally depends on the administration route/method, on the size and/or on the weight of the patient, on the detection technique used, and on the nature of the accessory moiety which may be associated therewith. As already mentioned herein, the patient preferably is a human.

The pharmaceutical composition of the invention may include a surfactant, a bulking agent, a stabilizer, a preservative, and a buffer, e.g., a buffer for adjusting the pH of the composition. In some embodiments, the formulation is a liquid formulation, a lyophilized formulation, a reconstituted lyophilized formulation, an aerosol formulation, or a bulk storage formulation (e.g., frozen bulk storage formulation).

"Therapeutically effective amount" means an amount of a compound that, when administered to human for treating a disease, is sufficient to achieve control of the disease. For example, the term "therapeutically effective amount" as used herein refers to the amount or quantity of a drug or pharmaceutically active substance which is sufficient to elicit the required or desired therapeutic response, or in other words, the amount which is sufficient to elicit an appreciable biological response when administered to a patient.

By providing the amino acid sequence of the nanobody of the invention, one skilled in the art is capable of producing the nanobodies according to the invention and described herein, by conventional techniques for producing polypeptides. For example, they may be synthesized by using a well-known synthesis method in a solid phase.

Alternatively, the nanobodies according to the invention may be synthesized with recombinant DNA techniques well known one skilled in the art (Maniatis et al. (1982) Molecular Cloning: a laboratory manual, Cold Spring Harbor Laboratories, NY, 51-54 and 412-430). For example, they may be obtained as DNA expression products after incorporating DNA sequences coding for the polypeptide of interest in expression vectors and introducing these vectors in suitable prokaryotic or eukaryotic hosts which will express the polypeptide of interest, from which they may then be isolated by using techniques well known to one skilled in the art. Accordingly, the invention also concerns a vector comprising a nucleic acid coding for the polypeptides and nanobodies as disclosed herein, as well as a transfected, infected or transformed cell with such a nucleic acid or vector.

Further advantages follow from the description of the embodiments and the attached drawings. All publications, patent applications, patents, and other references mentioned herein are incorporated by reference in their entirety.

Further, it is noted that, as used herein, the articles "a" and "an" refer to exactly (or only) one or to more than one (e.g., to at least one) of the grammatical object of the article.

The term "or" is used herein to mean, and is used interchangeably with, the term "and/or", unless context clearly indicates otherwise.

The terms "proteins" and "polypeptides" are used interchangeably herein, as well as the terms "nanobody" and "single domain antibody".

Also, the terms "about" and "approximately" and "substantially" shall generally mean an acceptable degree of error for the quantity measured given the nature or precision of the measurements. Exemplary degrees of error are within 20 percent (%), typically, within 10%, and more typically, within 5% of a given value or range of values.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description.

### BRIEF DESCRIPTION OF THE DRAWINGS

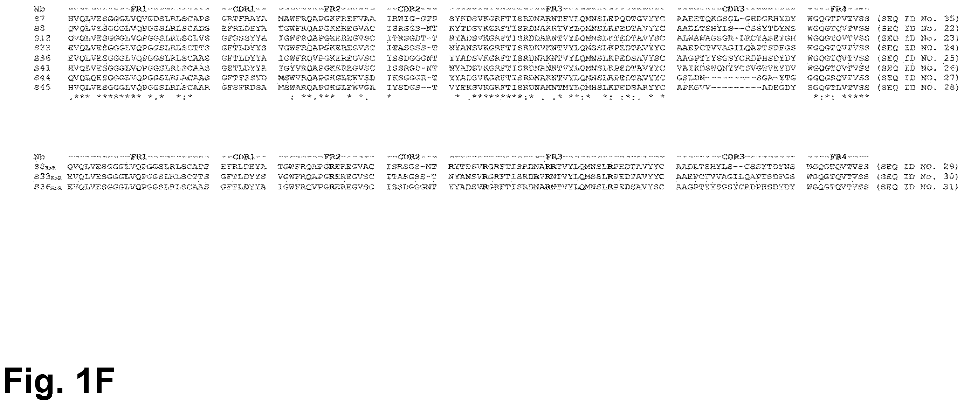

**Fig. 1** shows results for the biochemical characterization of hSIRPα nanobodies ("Nbs"): **(A)** shows the amino acid (aa) sequences of the complementarity determining region (CDR) 3 from 7 unique hSIRPα Nbs identified by a bidirectional screening strategy. Nbs S8, S12, S33 and S36 were selected against full length hSIRPα and Nbs S41, S44 and S45 against domain 1 of hSIRPα (hSIRPaD1); **(B)** shows the results of experiments concerning the recombinant expression and purification of hSIRPα Nbs using immobilized metal affinity chromatography (IMAC) and size exclusion chromatography (SEC). Coomassie staining of purified Nbs is **shown;(C)** shows the results of a stability analysis using nano-differential scanning fluorimetry (nanoDSF) displaying fluorescence ratio (350 nm/330 nm) and light intensity loss due to scattering shown as first derivative (upper panel) as well as biolayer interferometry (BLI)-based affinity measurements exemplarily shown for Nb S36. Biotinylated hSIRPα was immobilized on streptavidin biosensors. Kinetic measurements were performed using four concentrations of purified Nbs ranging from 0.625 nM to 5 nM (displayed with gradually lighter shades of color). The binding affinity (K_{D}) was calculated from global 1:1 fits shown as dashed lines (bottom panel); **(D)** shows a summary table of melting (T_{M}) and aggregation (T_{Agg}) temperature and affinities (K_{D}), association (*k*ₒₙ), and dissociation constants (*k*_{off}) determined for all selected hSIRPα Nbs; and (**E**) shows the staining results for representative images of hSIRPα and GFP expressing U2OS cells stained with hSIRPα Nbs. Images show individual Nb staining detected with anti-VHH-Cy5 (red), intracellular IRES derived GFP signal (green), nuclei staining (Hoechst, blue) und merged signals; scale bar: 50 µm; and (F) shows the full length (top) of 8 exemplary nanobodies according to the invention (designated in the figure with S7, S8, S12, S33, S36, S41, S44 and S45; also the respective CDRs and framework regions (FR) are indicated, respectively. Also shown are variants (bottom) of S8, S33 and S36, carrying amino acid exchanges (lysine to arginine; bold) in FR 2 and FR3, respectively (corresponding to SEQ ID Nos. 29, 30 and 31, respectively).

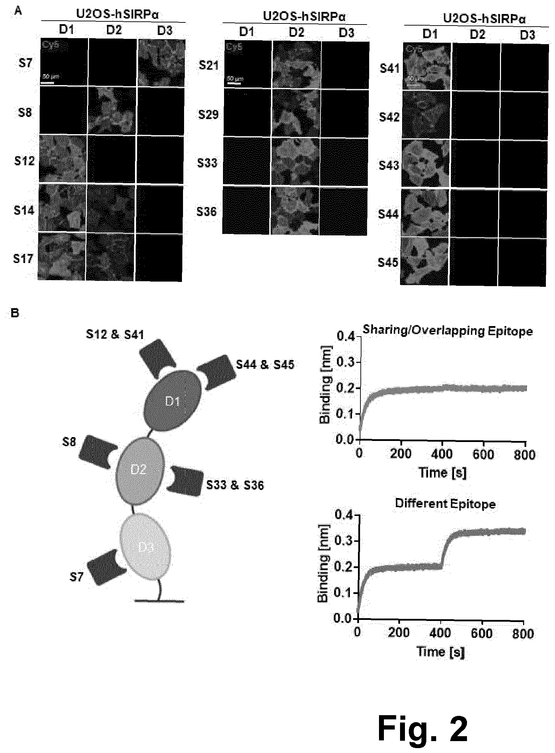

**Fig. 2** shows the results of the binding epitope characterization of hSIRPα nanobodies: (**A)** Domaine mapping analysis by immunofluorescence staining with hSIRPα Nbs on U2OS cells either displaying human hSIRPα domain 1 (D1), domain 2 (D2) or domain 3 (D3) at their surface. Representative images of live cells stained with individual Nbs detected with anti-VHH-Cy5 are shown; scale bar: 50 µm; (**B)** Epitope binning analysis of hSIRPα Nbs by BLI. Graphical summary of epitope binning analysis on the different hSIRPα domains (left panel). Representative sensograms of combinatorial Nb binding to recombinant hSIRPα on sharing/overlapping epitopes or on different epitopes (right panel).

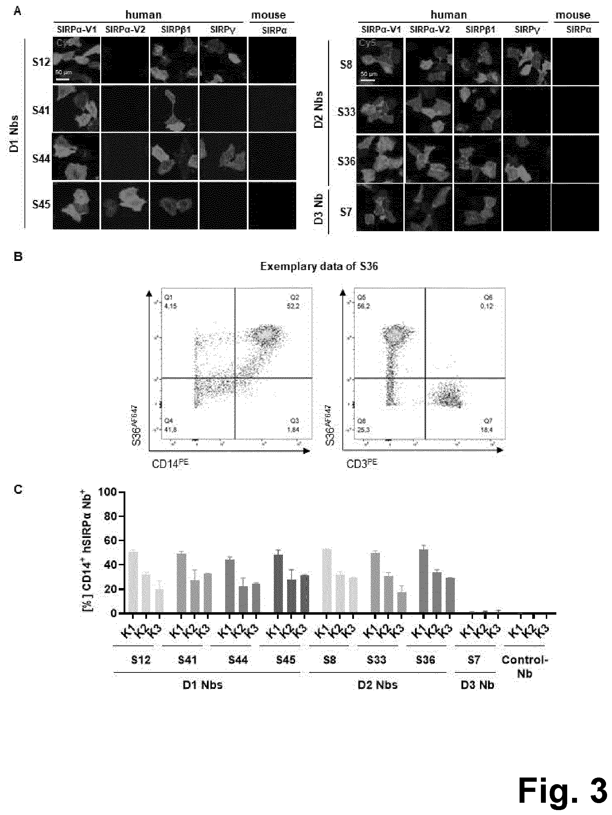

**Fig. 3** shows the results of experiments regarding the cross-reactivity and binding specificity of hSIRPα nanobodies: (**A)** Cross-reactivity analysis of hSIRPα Nbs by immunofluorescence staining on U2OS cells displaying hSIRPα-V1, -V2, hSIRPβ1, hSIRPγ or mouse SIRPα at their surface. Representative images of live cells stained with individual Nbs detected with anti-VHH-Cy5 are shown; scale bar: 50 µm; (**B)** Flow cytometry analysis (FACS) of human peripheral blood mononuclear cells (PBMCs) stained with fluorescently labeled hSIRPα Nbs (AlexaFluor 647, AF647). As example the flow cytometry plots of Nb S36 staining on CD14⁺ and CD3⁺ PBMC populations derived from human donor K1 are shown; (**C)** Flow cytometry analysis of hSIRPα Nbs staining CD14⁺ PBMCs of three different human donors (K1, K2, K3). Data are presented as mean ± SD of three technical replicates.

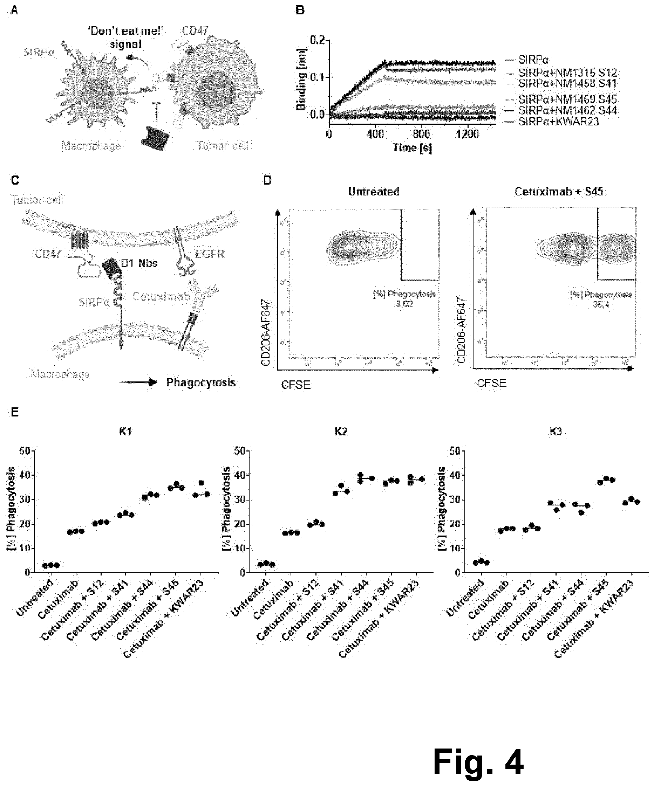

**Fig. 4** shows the results of experiments determining the potential of hSIRPαd1 nanobodies to augment phagocytosis of tumor cells: (**A)** Graphical illustration of hSIRPα/hCD47 interaction leading to suppression of macrophage-mediated phagocytosis of tumor cells; (**B)** Competition analysis of hSIRPα-binding to hCD47 in the presence of hSIRPαD1 Nbs (S12, S41, S44, S45) by BLI. Biotinylated hCD47 was immobilized on streptavidin biosensors and a mixture of 20 nM hSIRPα and 250 nM of hSIRPαD1 Nbs or 5 nM of KWAR23 were applied to elucidate potential inhibition of hSIRPα binding to hCD47; (**C)** Schematic illustration of macrophage-mediated phagocytosis of tumor cells by hSIRPaD1 Nbs and tumor-opsonizing antibodies (e.g., the anti-EGFR antibody cetuximab); (**D)** Phagocytosis of carboxyfluorescein diacetate succinimidyl ester (CFSE) labeled DLD-1 cells by human monocyte-derived macrophages. Shown is a representative flow cytometry plot of the phagocytosis assay of untreated and combinatorial treatment of cetuximab and hSIRPα Nb S45 with donor K1 derived macrophages; and (**E)** Quantitative analysis of the phagocytosis assay. Shown are percent of phagocytosis of CFSE-labeled DLD-1 cells analyzed for macrophages derived from three different donors (K1, K2, K3) either left untreated, upon addition of cetuximab only or following a combinatorial treatment with cetuximab with Nbs S12, S41, S44, S45 or antibody KWAR23 as positive control. Data are presented as mean ± SD of three technical replicates.

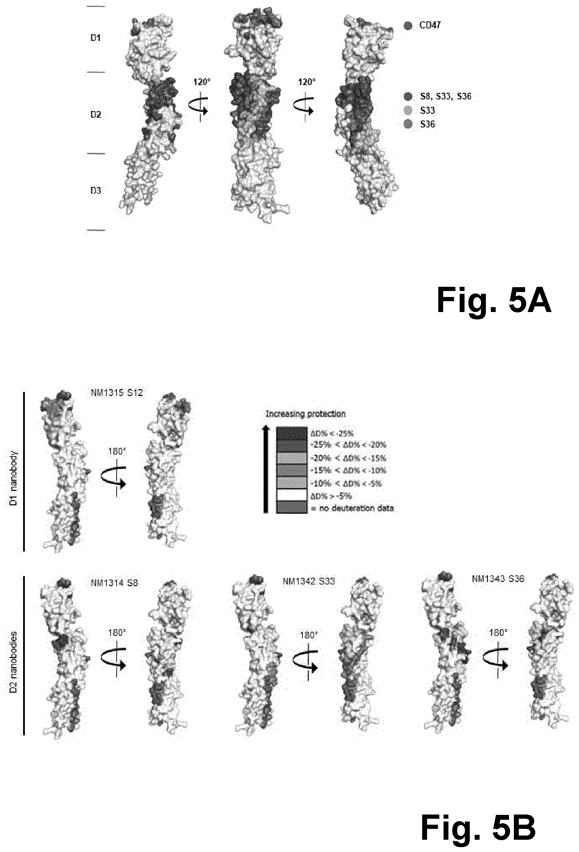

**Fig. 5** shows the results of the hydrogen-deuterium exchange mass spectrometry (HDX-MS) epitope mapping analysis/the localization of hSiRPαD2 nanobodies: (**A**) shows the localization of the binding epitopes for a surface structure model of hSIRPα (PDB 2wng) with epitope mapping results of Nbs S8, S33 and S36. Different dark shading highlights the amino acid residues protected by the individual Nbs or residues of hSIRPα involved in the hSIRPα/hCD47 interaction site; (**B**) shows a more detailed analysis of a surface structure model of hSIRPα (PDB 2wng) showing results of hydrogen-deuterium exchange followed by mass spectrometry (HDX-MS)-based epitope mapping; displayed are the results of individual hSIRPα epitopes protected upon binding of hSiRPαD2 Nbs S8, S33 and S36 and are highlighted in different colors indicating the strength of protection (%ΔD).

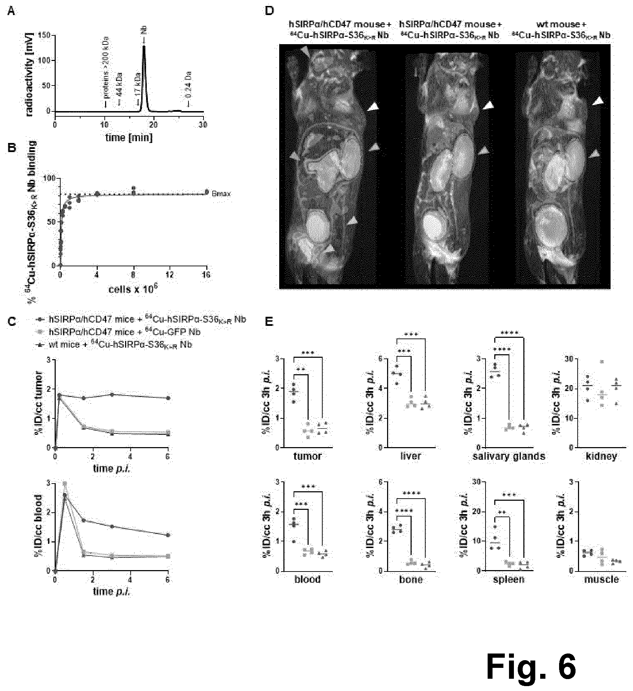

**Fig. 6** shows the results of PET imaging with ⁶⁴Cu-hSIRPα-S36_{K>R}nanobodies: (**A**) Radiochemical purity of ⁶⁴Cu-hSIRPα-S36_{K>R} Nb was assessed using high performance liquid chromatography; (**B**) Antigen excess binding assay to determine the maximum binding (Bmax) of ⁶⁴Cu-hSIRPα-S36_{K>R} Nb, referred to as immunoreactive fraction. ⁶⁴Cu-hSIRPα-S36_{K>R} Nb (1 ng) was applied to an increasing number of HT1080-hSiRPα cells (three technical replicates) and binding curves were analyzed using the one-site nonlinear regression model; (**C**) Quantification of ⁶⁴Cu-hSIRPα-S36_{K>R} Nb tumor and blood uptake over 6 h post injection revealed increased ⁶⁴Cu-hSIRPα-S36_{K>R} Nb accumulation compared to the control groups with control Nb or in wt; (**D**) Representative fused MIP (maximum intensity projection) PET/MR images of *s.c*. MC38-hCD47 colon carcinoma bearing hSIRPα/hCD47 mice 3h post ⁶⁴Cu-hSIRPα-S36_{K>R} Nb injection showed higher PET signal in hSIRPα expressing myeloid cell-rich organs compared to both control groups. Sites with increased ⁶⁴Cu-hSIRPα-S36_{K>R} Nb uptake are marked by colored arrows indicating the tumor (white), spleen (orange), bone (blue), salivary glands (purple), kidneys (green), and liver (red); and (**E**) Quantification of ⁶⁴Cu-hSIRPα-S36_{K>R} Nb revealed significantly higher tracer in hSIRPα expressing myeloid cell-rich organs. High accumulation was also detected in sites of excretion, namely kidney and liver. One-way ANOVA was performed for multiple comparisons using Tukey as a post hoc test (mean and SEM). A value of p < 0.05 was considered statistically significant and marked as * for p < 0.05, ** for p < 0.01, and *** for p < 0.001.

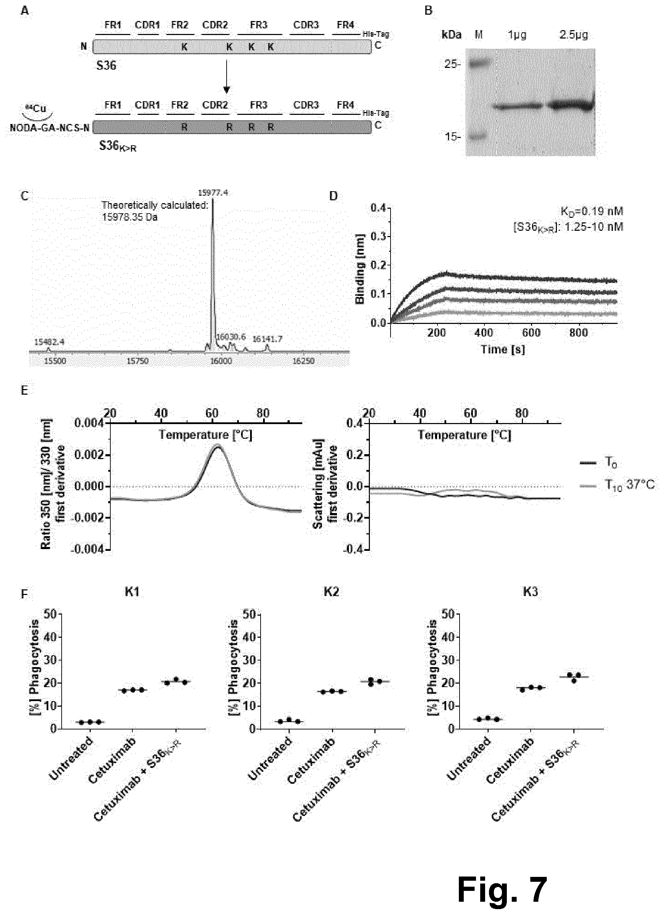

**Fig. 7** shows the results of a detailed characterization of the sequence optimized hSIRPα-S36_{K>R} nanobody for site-specific chelator conjugation: (**A**) Graphical illustration of sequence optimization of hSIRPα-S36 Nb (S36_{K>R}) by changing lysin (K) to arginine (R) residues for site-specific chelator (p-NCS-benzyl-NODA-GA) conjugation; (**B)** Expression and purification of hSIRPα-S36_{K>R} Nb using immobilized metal affinity chromatography (IMAC) and size exclusion chromatography (SEC). Coomassie staining of 1 µg and 2.5 µg of purified and conjugated hSIRPα-S36_{K>R} Nb is shown; (**C)** Confirmation of identity and integrity by mass spectrometric (MS) analysis of chelator conjugated hSiRPα-S36_{K>R} Nb (theoretically calculated molecular weight of 15978.35 Da); (**D)** BLI-based affinity measurements of chelator conjugated hSIRPα-S36_{K>R} Nb. Biotinylated hSIRPα was immobilized on streptavidin biosensors. Kinetic measurements were performed by using four concentrations of purified Nbs ranging from 1.25 nM to 10 nM; (**E)** Stability analysis of chelator conjugated hSIRPα-S36_{K>R} Nb by nanoDSF as fluorescence ratios (350 nm/330 nm) and light intensity loss due to scattering illustrated as first derivative before (T₀) and after 10 days of accelerated aging at 37°C (T₁₀); and (**F)** Phagocytosis of DLD-1 cells by human monocyte-derived macrophages treated with anti-EGFR cetuximab and chelator conjugated hSIRPα-S36_{K>R} Nb. Analysis of phagocytosis of hSIRPα-S36_{K>R} Nb in combination with cetuximab of three different donors (K1, K2, K3). Data are presented as mean ± SD of three technical replicates.

### DETAILED DESCRIPTION OF PREFERRED EMBODIMENTS

Within the present invention, a set of human SIRPα-specific nanobodies (in the following also: "Nbs") was generated, their binding properties on different recombinant SIRP-proteins (hSIRPβ1, hSIRPγ, and hSIRPα polymorphisms hSIRPα-V1 and hSIRPα-V2; and murine SIRPα) were examined, and their epitopes with sub-domain resolution were determined. Also binding studies of the hSIRPα nanobodies to human myeloid cells were performed, and their potential to functionally block the interaction to hCD47 was assessed. Also, epitope determination of the hSIRPα nanobodies as tracers for *in vivo* PET imaging was performed, as well as a site-directed functionalization of the hSIRPα-S36 nanobody for *in vivo* imaging. Further, by means of PET/MR imaging with a labeled nanobody (⁶⁴Cu-hSIRPα-S36_{K>R}), the distribution of SIRPα+ cells by non-invasive *in vivo* PET could be visualized and monitored.

### 1. Materials and Methods

### Expression constructs

DNA coding for hSiRPαV1 (GenBank accession: NM_001040022.1) and hSiRPαV2 (GenBank accession: D86043.1) were synthesized and cloned into Nhel and EcoRI site of pcDNA3.1(+) (GenScript Biotech). The vector backbone was adapted by cutting with EcoRI and BstBI and insertion of DNA comprising an internal ribosomal entry site (IRES) and genes for GFP and Blasticidin S deaminase from the expression construct described in (Traenkle, et al., "Single-domain antibodies for targeting, detection and in vivo imaging of human CD4+ cells", Front. Immunol. (2021) 12:799910). For the generation of hSIRPα expression constructs comprising Ig-like V-type domain (D1, aa 31-146), Ig-like C1-type 1 (D2, aa147-252) and Ig-like C1-type 2 (D3, aa253-348), of UniProtKB P13987 were genetically fused N-terminally to aa1-26 of huCD59 (UniProtKB P13987) and SPOT-Tag (see Virant et al., "A peptide tag-specific nanobody enables high-quality labeling for dSTORM imaging", Nature Communications (2018) 9:930) and C-terminally to aa91-128 of huCD59 and cloned into Bglll and Notl sites of pEGFPN2 expression vector. huCD59 sequences of the expressed fusion protein causes both translocation to the endoplasmatic reticulum and GPI anchoring of the protein at the plasma membrane. DNA encoding for hSIRPβ and hSIRPγ were purchased from addgene (Plasmid #116790) and Sino Biological (Catalog Number HG16111-NH) and subcloned into Nhel and EcoRI sites of expression vector used for hSIRPα variants. Expression vector for murine SIRPα was generated based on reference sequence NM_007547.4 and includes SPOT-Tag subsequent to signal peptide (aa 1-31). To generate the respective expression construct, cDNA was cloned into Kpnl and Xbal restriction sites of pCMV3-C-FLAG vector.

### Cell culture, transfection, stable cell line generation

U2OS, DLD-1, and HT-1080 cells (ATCC) were cultivated according to standard protocols in media containing DMEM (Thermo Fisher Scientific) or RPMI (Thermo Fisher Scientific), respectively supplemented with 10% (v/v) FBS (Thermo Fisher Scientific) and penicillin/streptomycin (Thermo Fisher Scientific) at 37°C and 5% CO₂ atmosphere in a humidified chamber and passaged using 0.05% trypsin-EDTA (Thermo Fisher Scientific). For transfection, Lipofectamine 2000 (Thermo Fisher Scientific) was used according to the manufacturer's protocol. To generate cells stably expressing hSIRPα, selection pressure was applied 24 h after transfection with 5 µg/ml Blasticidin S (Sigma Aldrich) for a period of two weeks, followed by single cell separation. Finally, individual clones were analyzed for hSIRPα expression.

### Cell Isolation

PBMCs were isolated as described previously (Traenkle etal., 2021). In brief, fresh blood was obtained from healthy volunteers and PBMCs were isolated by density gradient centrifugation with Biocoll separation solution (Biochrom) and frozen in heat-inactivated FBS (Capricorn Scientific, Germany) containing 10% dimethyl sulfoxide (DMSO; Merck).

### Nanobody library generation

For alpaca immunization and Nb library generation, a similar protocol as previously described was performed (Maier et al., "Real-time analysis of epithelial-mesen-chymal transition using fluorescent single-domain antibodies", Sci. Rep. (2015) 5:1-13; Traenkle et al., "Monitoring Interactions and Dynamics of Endogenous Beta-catenin With Intracellular Nanobodies in Living Cells", Mol. Cell. Prot. (2015) 14(3):707-723). Briefly, two alpacas (*Vicugna pacos*) were immunized with the extracellular portion of hSIRPα (aa31-370) produced in HEK293 cells (Acrobiosystems) with the approval of the Government of Upper Bavaria (approval number: 55.2-1-54-2532.0-80-14). After an initial vaccination with 560 µg, animals received five boost injections of 280 µg hSIRPα every two weeks. Finally, 91 days after initial immunization, -100 ml of blood was collected, and lymphocytes were isolated by Ficoll gradient centrifugation with lymphocyte separation medium (PAA Laboratories GmbH). To obtain cDNA, total RNA was extracted using TRI-zol (Life Technologies), followed by mRNA transcription using the First-Strand cDNA Synthesis Kit (GE Healthcare). The Nb repertoire was isolated in three subsequent PCR reactions using the following primer combinations: (1) CALL001 and CALL002, (2) forward primers FR1-1, FR1-2, FR1-3, FR1-4, and reverse primer CALL002, and (3) forward primers FR1-ext1 and FR1-ext2 and reverse primers FR4-1, FR4-2, FR4-3, FR4-4, FR4-5, and FR4-6 introducing Sfil and Notl restriction sites. Finally, the amplified Nb library was cloned into the pHEN4 phagemid vector (Arababi Ghahroud et al., "Selection and identification of single domain antibody fragments from camel heavy-chain antibodies" , FEBS letters (1997) 414(3):521-6) using the Sfil/Notl sites.

### Nanobody screening

For the selection of hSIRPα-specific Nbs, two consecutive phage enrichment rounds either with immobilized hSIRPα or hSIRPαD1 were performed. To generate Nb-presenting phages, TG1 cells comprising the Nb-library in pHEN4 were infected with the M13K07 helper phage. In each panning round 1 × 10¹¹ phages were applied to streptavidin or neutravidin plates (Thermo Fisher Scientific) coated with biotinylated antigen (5 µg/ml). For biotinylation purified antigen (Acrobiosystems) was reacted with Sulfo-NHS-LC-LC-Biotin (Thermo Fisher Scientific) in 5 molar excess at ambient temperature for 30 min. Excess of biotin was removed by size exclusion chromatography using Zeba^{™} Spin Desalting Columns 7K MWCO 0.5 ml (Thermo Fisher Scientific) according to manufacturer's protocol. Blocking of antigen and phage was performed alternatively with 5% milk or BSA in PBS-T and as the number of panning rounds increased, the wash stringency with PBS-T was intensified. Bound phages were eluted in 100 mM triethylamine, TEA (pH10.0), followed by immediate neutralization with 1 M Tris/HCl pH 7.4. Exponentially growing TG1 cells were infected with eluted phages and spread on selection plates for subsequent selection rounds. In each round, antigen-specific enrichment was monitored by counting colony forming units (CFUs).

### Whole-cell phage ELISA

For the monoclonal phage ELISA individual clones were picked and phage production was induced as described above. Moreover, 96-well cell culture plates (Corning) were coated with poly-L-lysine (Sigma Aldrich) and once washed with H₂O. U2OS-wt and U2OS overexpressing hSIRPα (U2OS-hSIRPα) or hSIRPαD1 (U2OS-hSIRPαD1) were plated at 2 × 10⁴ cells per well in 100 µl and grown overnight. Next day, 70 µl of phage supernatant was added to each cell type and incubated at 4°C for 3 h. Cells were washed 5 × with 5% FBS in PBS, followed by adding the M13-HRP-labeled detection antibody (Progen, 1:2000 Dilution) for 1 h, washed 3 × with 5% FBS in PBS. Finally, Onestep ultra TMB 32048 ELISA substrate (Thermo Fisher Scientific) was added to each well and incubated until color change was visible before stopping the reaction with 100 µl of 1 M H₂SO₄. For detection, the Pherastar plate reader at 450 nm was applied and phage ELISA-positive clones were defined by a 2-fold signal above (wildtype) wt control cells.

### Protein expression and purification

hSIRPα Nbs were cloned into the pHEN6 vector (see Arbabi Hhahroudi *et al.,* 1997, above) and expressed in XL-1 as previously described (Maier *et al.,* 2015; see above; Wagner et al., "NeutrobodyPlex-monitoring SARS-CoV-2 neutralizing immune responses using nanobodies" EMBO reports (2021) 22:e52325). Sortase A pen-tamutant (eSrtA) in pET29 was a gift from David Liu (Addgene plasmid # 75144) and was expressed as published in (Chen et al., "A general strategy for the evolution of bond-forming enzymes using yeast display" Proc. Natl. Acad. Sci. (2011) 108:11399-11404). Expressed proteins were purified by immobilized metal affinity chromatography using a HisTrap^{FF} column followed by a size exclusion chromatography (SEC, Superdex 75) on an Aekta pure system (Cytiva). Quality of all purified proteins was analyzed via standard SDS-PAGE under denaturizing conditions (5 min, 95°C in 2x SDS-sample buffer containing 60 mM Tris/HCl, pH6.8; 2% (w/v) SDS; 5% (v/v) 2-mercaptoethanol, 10% (v/v) glycerol, 0.02% bromphenole blue). For protein visualization InstantBlue Coomassie (Expe-deon) staining or alternatively immunoblotting as published previously (Traenkle *et al.,* 2021; see above) were performed. Protein concentration was determined by NanoDrop ND100 spectrophotometer.

### Biolayer interferometry (BLI)

Analysis of binding kinetics of hSIRPα specific Nbs was performed using the Octet RED96e system (Sartorius) as per the manufacturer's recommendations. In brief, 5 µg/ml of biotinylated hSIRPα diluted in Octet buffer (PBS, 0.1% BSA, 0.02% Tween20) was immobilized on streptavidin coated biosensor tips (SA, Sartorius) for 40 s. In the association step, a dilution series of Nbs ranging from 0.625-320 nM were reacted for 240 s followed by dissociation in Octet buffer for 720 s. Every run was normalized to a reference run applying Octet buffer for association. Data were analyzed using the Octet Data Analysis HT 12.0 software applying the 1:1 ligand-binding model and global fitting. For epitope binning two consecutive association steps with different Nbs were performed. By analyzing the binding behavior of the second Nb, conclusions about shared epitopes were drawn. For the hCD47 competition assay hCD47 was biotinylated and immobilized on SA biosensors followed by the application of pre-mixed solutions containing hSIRPα (20 nM) and Nb (250 nM). As control the hCD47-competing Ab KWAR23 (5 nM) was used.

### Live-cell immunofluorescence

Stably expressing hSIRPα U2OS cells, U2OS wt or U2OS cells transiently expressing individual hSIRPα domains (D1-3) with SPOT-Tag or different hSIRP family members (hSIRPα-V1, hSIRPα-V2, hSIRPβ1, hSIRPy, murine SIRPα) were plated at -10,000 cells per well of a µClear 96-well plate (Greiner Bio One, cat. #655090) and cultivated overnight at standard conditions. For imaging, medium was replaced by live-cell visualization medium DMEMgfp-2 (Evrogen, cat. #MC102) supplemented with 10% FBS, 2 mM L-glutamine, 2 µg/ml Hoechst33258 (Sigma Aldrich) for nuclear staining. 1-100 nM of unlabeled hSIRPα Nbs in combination with 2.5 µg/ml anti-VHH secondary Cy5 Affini-Pure Goat Anti-Alpaca IgG (Jackson Immuno Research) were added and incubated for 1 h at 37°C. For control staining hSIRPα Ab PE (SE5A5, BioLegend) and bivSPOT-Nb labeled with AlexaFluor647 (AF647) were used. Images were acquired with a MetaXpress Micro XL system (Molecular Devices) at 20x or 40x magnification.

### Stability analysis

Stability analysis was performed by the Prometheus NT.48 (Nanotemper). In brief, freshly-thawed hSIRPα Nbs were diluted to 0.25 mg/ml and measurements were carried out at time point T₀ or after incubation for ten days at 37°C (T₁₀) using high-sensitivity capillaries. Thermal unfolding and aggregation of the Nbs was induced by the application of a thermal ramp of 20-95°C, while measuring fluorescence ratios (F350/F330) and light scattering. Via the PR. ThermControl v2.0.4 the melting (T_{M}) and aggregation (T_{Agg}) temperature was determined.

### Fluorescent labeling of Nanobodies

For sortase coupling 50 µM Nb, 250 µM sortase peptide (H-Gly-Gly-Gly-propyl-azide synthesized by Intavis AG) dissolved in sortase buffer (50 mM Tris, pH 7.5, and 150 mM NaCl) and 10 µM sortase were mixed in coupling buffer (sortase buffer with 10 mM CaCl₂) and incubated for 4 h at 4°C. To stop the reaction and remove uncoupled Nb and sortase an IMAC was performed, followed by protein concentration and unreacted sortase peptide depletion using Amicon ultra-centrifugal filters MWCO 3 kDa. For fluorescent labeling, the SPAAC (strain-promoted azide-alkyne cycloaddition) click chemistry reaction was employed by incubating azide-coupled Nbs with 2-fold molar excess of DBCO-AF647 (Jena Bioscience) for 2 h at 25°C. Excess DBCO-AF647 was subsequently removed by dialysis (GeBAflex-tube, 6-8 kDa, Scienova). Finally, to deplete unlabeled Nb a hydrophobic interaction chromatography (HIC, HiTrap Butyl-S FF, Cytiva) was performed.

### Hydrogen-deuterium exchange

HDX-MS epitope mapping was performed as recently described (Gramlich et al., "A Novel PNGase Rc for Improved Protein N-Deglycosylation in Bioanalytics and Hydrogen-Deuterium Exchange Coupled With Mass Spectrometry Epitope Mapping under Challenging Conditions", Analytical Chemistry (2022) 94:9863-9871). In brief, 5 µL hSIRPα (42 µM) was incubated with either 2.5 µL PBS or a specific hSIRPα Nb, S7 (90 µM), S8 (103 µM), S33 (145 µM) or S36 (78 µM). After a 10 min pre-incubation at 25 °C, HDX was initiated by a 1:10 dilution in PBS (pH 7.4) prepared with D₂O (final labeling D₂O concentration = 90%). Aliquots of 15 µL were quenched after deuteration for 5 and 30 min by adding 15 µL ice-cold quenching solution (200 mM TCEP, 1.5% formic acid and 4 M guanidine HCl in 100 mM ammonium formate solution, pH 2.2) resulting in a final pH of 2.5. Samples were immediately snap frozen and stored at -80 °C until analysis. Non-deuterated control samples were processed using PBS prepared with H₂O. Each sample was prepared in independent technical replicates (n=3). Settled gel of immobilized pepsin (Thermo Fisher Scientific) was prepared by centrifugation of 60 µL 50% slurry (in ammonium formate solution pH 2.5) at 1,000 × g and 0 °C for 3 min. The supernatant was discarded, sample aliquots were thawed and added to the settled pepsin gel. The proteolysis was performed for 2 min in a water-ice bath. To improve sequence coverage near the N-glycosylation sites of hSIRPα, a post-proteolysis deglycosylation was performed using PNGase Rc (Gramlich *et al.,* 2022; see above). For this purpose, 5 µL PNGase Rc (4 µM) was added under a filter inlet (0.65 µm, Merck Millipore) and the proteolyzed sample was placed on the filter. Centrifugation at 1000 × g for 30 s at 0 °C removed the beads and initiated the deglycosylation of the peptides in the flow-though. Deglycosylation was carried out in a water-ice bath for an additional 2 min, and samples were analyzed by LC-MS as described in (Wagner *et al.,* 2021; see above).

Data analysis was performed as described in (Wagner *et al.,* 2021; see above). HDX data were obtained for ≥83% of the hSIRPα sequence. The deuterium uptake of each peptide was normalized to the maximal exchangeable protons of the backbone. The deuteration was compared between hSIRPα alone and in complex. A peptide was considered protected from HDX if the summed difference was ≥5%. A peptide was considered not protected if the summed HDX difference was ≤3%.

### Mass spectrometry

To confirm correct expression, integrity, and purity, chelator conjugated hSIRPα-S36_{K>R} was analyzed by mass spectrometry. Protein sample (5 µg) was diluted 1:3 with HisNaCl buffer (20 mM His, 140 mM NaCl, pH 6.0) and analyzed by liquid chromatography (HPLC) coupled to electrospray ionization (ESI) quadrupole time-of-flight (QTOF) MS. Sample (0.4 µg per injection) was desalted using reversed phase chromatography on a Dionex U3000 RSLC system (Thermo Scientific, Dreieich, Germany) using a Acquity BEH300 C4 column (1mm × 50mm, Waters, Eschborn, Germany) at 75°C and 150 µl/min flow rate applying a 11-min linear gradient with varying slopes. In detail, the gradient steps were applied as follows (min/% Eluent B): 0/5, 0.4/5, 2.55/30, 7/50, 7.5/99, 8/5, 8.75/99, 9.5/5, 10/99, 10.25/5 and 11/5. Eluent B was acetonitrile with 0.1% formic acid, and solvent A was water with 0.1% formic acid. To avoid contamination of the mass spectrometer with buffer salts, the HPLC eluate was directed into waste for the first 2 min. Continuous MS analysis was performed using a QTOF mass spectrometer (Maxis UHR-TOF; Bruker, Bremen, Germany) with an ESI source operating in positive ion mode. Spectra were taken in the mass range of 600-2000 m/z. External calibration was applied by infusion of tune mix via a syringe pump during a short time segment at the beginning of the run. Raw MS data were lock-mass corrected (at m/z 1221.9906) and further processed using Data Analysis 5.3 and MaxEnt Deconvolution software tools (Bruker).

### Flow cytometry

For flow cytometry analysis 200 000 cells per staining condition were used in FACS buffer: PBS containing 0.02% sodium azide, 2 mM EDTA, 2% (v/v) FBS (Thermo Fisher Scientific). Extracellular staining was performed with hSIRPα Nbs conjugated to AF647 (200 nM), hSIRPα Ab PE (SE5A5, BioLegend), CD3 Ab APC/Cy7 (HIT3a, BioLegend), CD14 Ab PE (HCD14, BioLegend), the dead cell marker Zombie Violet (BioLegend) or the respective unspecific fluorescently labeled Pep Nb (PEP-Nb_{AF647}) (Traenkle *et al.,* 2021; see above) and isotype control Abs (BioLegend) by incubation for 45 min at 4°C. Cells were washed three times with FACS buffer and data were acquired on the same day using a LSRFortessa^{™} flow cytometer (Becton Dickinson) equipped with the DIVA Software (Becton Dickinson). Final data analysis was performed using the FlowJo10^{®} software (Becton Dickinson).

### Macrophage-mediated antibody dependent cellular phagocytosis assay

CD14⁺ cells were purified from frozen PBMCs and CD14 positive selection (Miltenyi Biotec) according to manufacturers' protocols. Monocyte-derived macrophages (MDM) were generated by seeding three million CD14⁺ cells into one 6-well plate (NuncTM Thermo Fisher Scientific) in IMDM (Thermo Fisher Scientific) supplemented with 10% (v/v) fetal bovine serum (Thermo Fisher Scientific) and 50 ng/mL M-CSF (Miltenyi Biotec) and cultured for 7 to 9 days. Cells were detached from culture plates with Accutase^{®} (Sigma Aldrich). DLD-1 cells were labeled with CFSE Cell Division Tracker Kit (BioLegend) according to manufacturer's instructions. 100,000 DLD-1 cells and 50,000 MDMs were incubated in U-bottom 96-well plates (Corning) with hSIRPα Nbs (1 µM) or KWAR23 (100 nM) and Cetuximab (0.66 nM) (MedChemExpress) for 2 h at 37° C, followed by detachment of adherent cells from culture plates with Accutase^{®} (Sigma Aldrich). For flow cytometry, cells were incubated with CD206 Ab AF647 (clone 15-2, BioLegend) and dead cell marker Zombie Violet (BioLegend). Percent phagocytosis indicates the percentage of viable CD206⁺CFSE⁺ macrophages.

### Chelator conjugation and Radiolabeling

For chelator conjugation and radiolabeling with ⁶⁴Cu metal-free equipment and buffers pretreated with Chelex 100 (Sigma-Aldrich) were used. Nbs (100 µg) were reacted with 100 molar equivalents of p-NCS-benzyl-NODA-GA (CheMatech) in 0.2 M sodium bicarbonate pH 8.7 for 24 h at RT. Excess of chelator was removed by ultrafiltration (Amicon Ultra 0.5 ml, 3 kDa MWCO, Merck Millipore) using the same buffer conditions. For neutralization of [⁶⁴Cu]CuCl₂ (150 MBq in 0.1 M HCl) 1.5 volumes of 0.5 M ammonium acetate solution (pH 4.1) were added, resulting in a pH between 4 - 5. In the following, 50 µg of conjugate was applied to the solution and incubated at 35°C for 30 min. To quench the labeling reaction 20% diethylenetriaminepentaacetic acid (DTPA) solution in a 1:100 was added. Complete incorporation of the radioisotope was confirmed after each radiosynthesis by thin-layer chromatography (iTLC-SA; Agilent Technologies; mobile phase 0.1 M citric acid pH 5) and high-performance size exclusion chromatography (HPSEC; BioSep SEC-s2000, 300 × 7.8 mm, Phenomenex; mobile phase DPBS with 0.5 mM EDTA).

### In vitro radioimmunoassay

To determine the immunoreactive fraction (maximum binding, Bₘₐₓ), an increasing number of HT1080-hSiRPα cells were incubated in triplicates with 1 ng (2 MBq/µg) of ⁶⁴Cu-hSIRPα-S36_{K>R} Nb for 1 h at 37°C and washed twice with PBS/1% FCS. The remaining cell-bound radioactivity was measured using a Wizard² 2480 gamma counter (PerkinElmer Inc) and quantified as percentage of the total added activity.

### Mouse Models and PET imaging

Six-week-old female C57BL/6N wt mice were purchased from Charles River. C57BL/6 hSIRPα/ hCD47 KI (C57BL/6N^{CD47tm1.1(CD47)Geno;Sirpαtm2.1(SIRPA)Geno}) mice (hSIRPα/hCD47) were developed by genOway. For tumor cell inoculation, 1 × 10⁶ MC38 hCD47 KI cells or MC38 wt colon adenocarcinoma cells (both genOway), were resuspended in 100 µl PBS and were then subcutaneously injected into hSIRPα/hCD47or wt mice, respectively.

hSIRPα/hCD47 and wt mice were injected intravenously (*i.v*.) with 5 µg (-10 MBq) of ⁶⁴Cu-hSIRPα-S36_{K>R} Nbor ⁶⁴Cu-GFP Nb 9 days after tumor cell inoculation. Mice were anesthetized with 1.5% isoflurane in 100% oxygen during the scans. Ten-minute static PET scans were performed after 5 min, 90 min, 3 h and 6 h in a dedicated small-animal Inveon micro PET scanner (Siemens Healthineers, 600 s acquisition time). For anatomical information, sequential T2 TurboRARE MR images were acquired immediately after the PET scans on a small animal 7 T ClinScan magnetic resonance scanner (Bruker BioSpin GmbH). PET images were reconstructed using an ordered subset expectation maximization (OSEM3D) algorithm and analyzed with Inveon Research Workplace (Siemens Preclinical Solutions). The volumes of interest of each organ were defined based on anatomical MRI to acquire the corresponding PET tracer uptake within the tumor and organs of interest. The resulting values were decay-corrected and presented as percentage of injected dose per cubic centimeter (%ID/cc).

### Analyses, statistics, and graphical illustrations

Graph preparation and statistical analysis were performed using the GraphPad Prism Software (Version 9.0.0 or higher). Graphical illustrations were created with BioRender.com.

### 2. Results

### Selection of high-affinity anti-human SIRPα nanobodies

To generate Nbs against hSIRPα that can be used either as probes for diagnostic imaging or to modulate interaction with human CD47, an alpaca (*Vicugna pa*cos) was immunized with the recombinant extracellular portion of hSIRPα and a Nb phagemid library (2 × 10⁷ clones) was established representing the repertoire of variable heavy chain antibodies (VHHs or Nbs). This Nb phagemid library was subjected to phage display-based selection campaigns either against the entire extracellular portion or exclusively domain 1 (D1) of hSIRPα (hSIRPαD1) to direct the selection of Nbs blocking the hSIRPα/hCD47 interaction. Positive binders were identified in a whole-cell ELISA screen using cells stably expressing hSIRPα or hSIRPαD1. Sequencing of individual clones resulted in 14 unique hSIRPα Nbs with highly diverse complementarity determining regions (CDRs) 3 (**Figure 1A**). Nbs S7 to S36 were identified in the full-lengths hSIRPα screening, whereas Nbs S41 to S45 were selected to specifically bind hSIRPαD1. Individual Nbs were produced with moderate to high yields in *Escherichia coli* (*E. coli*) and isolated with high purity (**Figure 1B**). To assess folding stability, all Nbs were analyzed by differential scanning fluorimetry. For 12 out of the 14 Nb candidates, melting temperatures ranged from ~55°C to ~75°C without aggregation (**Figure 1C****, D; Supplementary** **Figure 1A**). Affinity measurements against recombinant hSIRPα by biolayer interferometry (BLI) revealed K_{D} values between -0.12 and -27 nM for 11 out of the 12 Nbs, whereas affinity determination for Nb S42 was not possible (**Figure 1C****, D; and data not shown**). To test, whether the selected Nbs also recognize plasma membrane-localized hSIRPα, immunofluorescence live-cell staining of U2OS cells stably expressing full-length hSIRPα was performed, which confirmed specific binding of all selected Nbs (**Figure 1E****; and data not shwon**).

### Domain mapping of hSIRPα Nbs

For the different intended uses of the Nbs, different domain specificities are favorable, as Nbs targeting hSIRPαD1 have a higher potential to block interaction with CD47, whereas Nbs targeting domain D2 or D3 (hSIRPαD2, hSIRPαD3) might be functionally inert, which is preferable for diagnostic approaches. Thus, U2OS cells expressing the individual domains of hSIRPα were used and the domain specificity of all selected Nbs was determined by immunofluorescence staining (**Figure 2A****, and data not shown**). Eight Nbs (S12, S14, S17, S41, S42, S43, S44, and S45) stained hSIRPaD1, whereas Nbs S14 and S17 additionally stained hSIRPαD2. Five Nbs (S8, S21, S29, S33 and S36) revealed specific binding to hSiRPαD2, whereas only Nb S7 stained cells expressing hSIRPαD3. Based on their respective production yield, stability, affinity, domain specificity and developability, Nbs S7, S8, S12, S33, S36, S41, S44 and S45 were selected for further characterization.

To determine the diversity of epitopes recognized by this subset in more detail, an epitope binning analysis was performed using BLI (**Figure 2B****; and data not shown**). Binning experiments were done by sequentially applying hSIRPαD1- or hSIRPαD2-targeting Nbs to hSIRPα immobilized at the sensor tip. The detection of a single association curve or stacked curves indicates shared epitopes or independent binding to separate epitopes, respectively. Based on the results, the Nbs according were grouped to shared or overlapping epitopes and two groups were found each for hSIRPαD1-targeting (Nb S12 & S41 and Nb S44 & S45) and hSIRPαD2-targeting Nbs (S8 and Nbs S33 & S36).

### Specificity of hSIRPα Nbs to allelic variants and closely related SIRP family members

hSIRPα belongs to the hSIRP family of immune receptors, which also includes the highly homologous activating receptor hSIRPβ1, also predominantly found on macrophages, and the decoy receptor hSIRPγ, which is expressed mainly on T cells (Matlung et al., "The CD47-SIRPα signaling axis as an innate immune checkpoint in cancer", Immunological reviews (2017) 276: 145-164). Moreover, with hSiRPαV1 and hSiRPαV2, two isoforms are expressed either homozygously (v1/v1 or v2/v2) or heterozy-gously (v1/v2), and the frequency of distribution varies between human subpopulations (Sim et al., "Discovery of high affinity, pan-allelic, and pan-mammalian reactive antibodies against the myeloid checkpoint receptor SIRPα", MAbs. Taylor & Francis (2019) pp. 1036-1052). To address the question of specificity, hSIRPβ1, hSIRPγ or the hSIRPα polymorphisms hSIRPα-V1 and hSIRPα-V2 were transiently expressed in U2OS cells and their recognition was analyzed by the selected hSIRPα Nbs by immunofluorescence staining. In addition, murine SIRPα was included to evaluate potential cross-reactivity between human and mouse SIRPα variants (**Figure 3A****; data not shown**). Imaging revealed that all Nbs recognized the homologous hSIRPβ1 whereas hSIRPγ was detected with Nbs S12 and S44 (both hSIRPαD1-targeting Nbs) as well as Nbs S8 and S36 (both hSIRPαD2-targeting Nbs). Furthermore, all hSIRPαD2- and D3-targeting Nbs recognized hSIRPα-V1 and -V2, whereas for the hSIRPαD1-targeting Nbs only S45 showed binding to both polymorphisms. Notably, none of the selected Nbs revealed any cross-reactivity towards murine SiRPα.

### Binding of hSIRPα Nbs to primary human myeloid cells

Next, it was evaluated whether the hSIRPα Nbs of the invention also recognize endogenously expressed hSIRPα. Therefore, flow cytometry analysis of peripheral blood mononuclear cells (PBMCs) from three different donors (K1-3) was performed. In addition to the monocyte/macrophage marker CD14, also the T cell marker CD3 was included to evaluate potential recognition of T cells by hSIRPγ-cross-reactive Nbs (**Figure 3B****).** All hSIRPα Nbs, with the exception of S7, stained comparable hSIRPα expression on CD14⁺ PBMCs from all donors tested (**Figure 3C**), whereas none of the Nbs stained CD3⁺ T cells **(data not shown).**

Considering the binary strategy to select hSIRPα Nbs (i) which are eligible to inhibit the hSIRPα/hCD47 interaction and (ii) as probes for PET-based *in vivo* imaging of myeloid cells, the identified Nbs were divided into two subgroups. In the following, hSIRPαD1-targeting Nbs S12, S41, S44, and S45 were further investigated with respect to their inhibitory properties, and hSIRPαD2-targeting Nbs S8, S33 and S36 for their applicability as *in vivo* imaging probes.

### hSIRPαD1 nanobodies functionally block the interaction to hCD47

To evaluate potential inhibition of the interaction between hSIRPα and hCD47 **(****Figure 4A****),** a competitive BLI-based binding assay was performed analyzing the binding of hSIRPα to immobilized CD47 in the presence or absence of hSIRPαD1-targeting Nbs. As control, the anti-human SIRPα-blocking antibody KWAR23 (Ring et al., "Anti-SIRPα antibody immunotherapy enhances neutrophil and macrophage antitumor activity", Proc. Nat. Sci. (2017) 114: E10578-E10585) was used. After addition of Nbs S44 or S45, the binding of hSIRPα to CD47 was inhibited to a similar extent as in the presence of KWAR23, whereas only partial blocking was observed for S41, while S12 showed no effect **(****Figure 4B****).** Next, the ability of hSIRPαD1-targeting Nbs to potentiate macrophage-mediated antibody-dependent cellular phagocytosis (ADCP) was tested **(****Figure 4C****).** To this end, human monocyte-derived macrophages (MDMs) isolated from three different donors (K1-3) were incubated with EGFR⁺ DLD-1 tumor cells preloaded with carboxyfluorescein diacetate succinimidyl ester (CFSE) in the presence of the opsonizing antibody cetuximab and hSIRPαD1-targeting Nbs or the KWAR23 antibody as positive control. As DLD-1 cells confer CFSE staining to macrophages upon phagocytosis, the degree of ADCP was determined based on CD206⁺CFSE⁺ cells by flow cytometry analysis **(****Figure 4D****).** For all donors tested, macrophages exhibited minimal phagocytosis of DLD-1 cells without treatment, whereas phagocytic activity was significantly increased upon addition of cetuximab. In the presence of the hSIRPα-blocking antibody KWAR23, phagocytosis was further induced, which is in line with previous findings (Ring *et al.,* 2017; see above). Similarly, the hSIRPα-blocking Nbs S44 and S45 but also the partially hSIRPα-blocking Nb S41 augmented ADCP in all three tested donors, whereas Nb S12 had no effect on macrophage-mediated phagocytosis **(****Figure 4E****).** From these results, it was concluded that Nbs S41, S44, and S45 not only block the interaction between hSIRPα and hCD47 *in vitro,* but also functionally enhance macrophage-mediated ADCP in combination with opsonizing antibodies, and thus represent promising candidates for further development as novel biologicals, e.g., for combinatory therapies of advanced malignancies.

### Epitope determination of hSIRPα Nbs as prospective tracers for in vivo PET imaging

In contrast to hSIRPα/hCD47 blocking binders, hSIRPα Nbs assigned as diagnostic tracers for non-invasive *in vivo* PET imaging should not exhibit hSIRPα/hCD47 blocking. For this, Nbs S8, S33 and S36 were selected, which bind to hSiRPαD2 and performed a detailed analysis of the recognized epitopes by hydrogen-deuterium exchange mass spectrometry (HDX-MS). The analysis showed that the selected Nbs recognized three-dimensional epitopes within hSIRPαD2, which are spatially distant from the hSIRPα/hCD47 interface **(****Figure 5A** and **5B**). In particular, S36 Nb showed the strongest deuteration protection (< -15%) for amino acid (aa) D162 - L186 of hSIRPα, whereas an additional lower protection was observed for the regions ranging from aa C139 - K152 and aa H201 - G206 (**Figure 5A** and **5B**). Based on the detailed epitope mapping, its strong binding affinity and good production yield, hSIRPα-S36 Nb was chosen as a first candidate for imaging experiments.

### Site-directed functionalization of hSIRPα-S36 Nb for in vivo imaging

For the application as a PET tracer, hSIRPα-S36 Nb must be modified by the addition of a chelator for radionuclide labeling. In contrast to conventional non-selective bioconjugation, recent efforts have been made to enable site-directed conjugation, e.g., by chemoenzymatic labeling based on sortase-mediated coupling (Massa et al., "Sortase A-mediated site-specific labeling of camelid single-domain antibody-fragments: a versatile strategy for multiple molecular imaging modalities", Contrast Media Mol Imaging (2016) 11: 328-339); Rashidian et al., "Predicting the response to CTLA-4 blockade by longitudinal noninvasive monitoring of CD8 T cells", Journal of Experimental Medicine (2017) 214: 2243-2255); Traenkle *et al.,* 2021; see above). Although this approach resulted in conjugated Nbs with high purity (Traenkle *et al.,* 2021; see above), the efficiencies are limited by the enzymatic reverse reaction. Alternatively, maleimide coupling of single solvent-exposed cysteine residues incorporated into the sequence can be employed. However, considering that the hSIRPα-S36 Nb contains two disulfide bonds, problems were anticipated due to the formation of intermolecular disulfide bonds during the reduction and oxidation steps required for this conjugation approach. For this reason, a novel protein engineering approach was conceived that enables site-specific chemical conjugation. First, the sequence of the original hSIRPα-S36 Nb was adapted by exchanging all four lysine residues with arginine (hSIRPα-S36_{K>R} Nb) (**Figure 7A**). Subsequently, the chelator was covalently conjugated via isothiocyanate (p-NCS-benzyl-NODA-GA) to the single remaining primary NH₂-group at the N-terminus **(****Figure 7A****).** The hSIRPα-S36_{K>R} Nb was produced with comparable yield and purity to the original version in *E.coli* **(****Figure 7B****).** Site-specific chelator conjugation with an efficiency of -96% was confirmed by mass spectrometry **(****Figure 7C****).** Next, it was tested whether these changes affected the binding properties. BLI data showed that the chelator-conjugated hSIRPα-S36_{K>R} Nb retained highly similar binding affinities to recombinant hSIRPα compared to the original version **(****Figure 7D****).** Furthermore, stability analysis revealed thermal stability up to -62 °C without aggregation. Also, after accelerated aging at 37°C for 10 days, no significant differences were detected compared to baseline **(****Figure 7E****).** Finally, to investigate non-modulatory properties, the hSIRPα-S36_{K>R} Nb was examined in the macrophage-dependent phagocytosis assay as described above. As expected, compared with cetuximab mono-treatment alone, a negligible extent of macrophage phagocytosis was observed in the presence of the hSIRPα-S36_{K>R} Nb **(****Figure 7F****).** From these results, it was concluded that the modified SlRPαD2-targeting Nb S36, eligible for site-directed chelator conjugation by targeted sequence optimization, represents a promising candidate for non-invasive *in vivo* PET imaging of SIRPα expression signatures.

### PET/MR imaging with ⁶⁴Cu-hSIRPα-S36_{K>R} Nb

Currently, suitable rodent models for *in vivo* application of the hSIRP-S36_{K>R} Nb are commercially not available. So far, human-specific hSIRPα antibodies have been tested, for example, in hSIRPα KI SRG mice, which, however, lack hCD47 (Ring *et al.,* 2017; see above). Therefore, here for the first time a newly developed hSIRPα/hCD47 KI mouse model (hSIRPα/hCD47 mice) was employed to perform *in vivo* PET imaging with the hSIRPα-S36_{K>R} Nb.

For non-invasive *in vivo* validation, the hSIRPα-S36_{K>R} Nb and a nonspecific GFP Nb were radiolabeled with ⁶⁴Cu. Radiolabeling yielded high radiochemical purities of ≥95% (**Figure 6A**) and an immunoreactivity of ~82% (Bₘₐₓ) of the ⁶⁴Cu-labeled hSIRPα-S36_{K>R} Nb (⁶⁴Cu-hSIRPα-S36_{K>R} Nb) to HT1080 hSIRPα KI (HT1080-hSIRPα) cells *in vitro* (**Figure 6B**). To visualize the distribution of hSIRPα-positive cells in a tumor-relevant system, MC38 adenocarcinoma cells were subcutaneously (*s*.*c*.) injected either in hSIRPα/hCD47 or C57BL/6 wildtype (wt) mice. Nine days after tumor inoculation, ⁶⁴Cu-hSIRPα-S36_{K>R} Nb was intravenously (*i.v.*) injected into MC38 adenocarcinoma-bearing hSIRPα/hCD47 or wt mice. As additional control, the non-hSIRPα-specific ⁶⁴Cu-GFP Nb was injected in tumor-bearing hSIRPα/hCD47 mice.

Non-invasive *in vivo* PET/MR imaging revealed a strongly enhanced ⁶⁴Cu-hSIRPα-S36_{K>R} Nb accumulation in the tumors of hSIRPα/hCD47 mice within the first minutes after injection, that remained an identical high level over 6 hours, whereas a rapid tracer clearance occurred in the tumors and the blood of both control groups, the control ⁶⁴Cu-GFP Nb injected hSIRPα/hCD47 mice and ⁶⁴Cu-hSIRPα-S36_{K>R} Nb injected wt mice (**Figure 6C**). Importantly, ⁶⁴Cu-hSIRPα-S36_{K>R} Nb-injected in hSIRPα/hCD47 mice exhibited a constantly higher PET signal in the blood over time, indicating a specific binding to circulating hSIRPα⁺ myeloid cells (**Figure 6C**). Quantification of the PET images 3 h after injection revealed a significantly higher uptake in the tumors of hSIRPα/hCD47 mice (1.89 ± 0.09 %ID/cc) compared to wt mice (0.60 ± 0.05 %ID/cc) and to ⁶⁴Cu-GFP Nb injected hSIRPα/hCD47 mice (0.57 ± 0.05 %ID/cc) (**Figure 6C-E**). Furthermore, a ~7-fold enhanced uptake in the spleen was observed, a ~2-fold enhanced uptake in the blood and liver, and a ~3-fold enhanced uptake in the salivary glands and bone of hSIRPα/hCD47 mice compared to the two control groups (**Figure 6D****, E**). No significant differences in ⁶⁴Cu-hSIRPα-S36_{K>R} Nb accumulation were identified in the kidneys and the muscle tissue between the ⁶⁴Cu-hSIRPα-S36_{K>R} Nb injected hSIRPα/hCD47 mice and the two described control groups (**Figure 6D****, E**). In summary, these results demonstrate the applicability of the novel ⁶⁴Cu-hSIRPα-S36_{K>R} Nb-based PET-tracer to visualize and monitor the distribution of SIRPα⁺ cells by non-invasive *in vivo* PET.

### 3. Summarizing Discussion

Immunotherapies have considerably improved the therapeutic options for cancer patients. However, achieving complete remission with durable responses remains for a large number of patients a major challenge. This highlights the urgent need for new classes and combinations of specific individualized immunotherapeutics and diagnostic tools for targeted patient stratification and treatment monitoring. Since myeloid cells, such as monocytes, dendritic cells, granulocytes and in particular macrophages frequently infiltrate tumors, modulate tumor angiogenesis, promote metastasis, and have been associated with tumor resistance to chemotherapy and immune checkpoint blockade, their characteristic marker, the immune checkpoint SIRPα has been chosen in the present invention as promising a strategy for the treatment of advanced cancers.

Beside serving as therapeutic target, SIRPα represents also a biomarker, which can be used stratify patients concerning myeloid cell expression patterns and in addition to track the migration and dynamics of myeloid cells in the context of cancer and cancer immunotherapy.

Thus, in the present invention, human SIRPα-specific Nbs were developed, either as modulatory biologics blocking the hSIRPα/hCD47 axis or for monitoring TAMs as the most abundant myeloid cell type within the TME. Following a binary screening strategy and subsequent in-depth biochemical characterization with the Nb S41, S44 and S45 the first hSIRPα-specific Nbs were identified, which exclusively bind the D1 domain of hSIRPα and thus selectively block the interaction with CD47. *In vitro* phagocytosis studies with human macrophages and cancer cells revealed that in combination with the tumor-opsonizing antibody cetuximab, both monovalent Nbs S44 and S45 functionally enhance ADCP to a similar extent as recently reported for the full-length hSIRPα-blocking antibody KWAR23 (Ring *et al.,* 2017; see above). In particular, the selectivity of Nb S45 for binding to hSIRPα but not to hSIRPγ is advantageous, as recent data showed that nonselective hSIRPα/hSIRPγ blockade can impair T cell activation, proliferation, and endothelial transmigration (Gauttier et al., "Selective SIRPα blockade reverses tumor T cell exclusion and overcomes cancer immunotherapy resistance", The Journal of clinical investigation (2020) 130: 6109-6123).

In this connection, it is noted, that blocking efficacies of the inhibitory hSIRPα-specific Nbs can be further improved by establishing bivalent or biparatopic formats as shown previously (see, e.g., Virant *et al.,* 2018; Wagner *et al.,* 2022; see both above).

Alternatively, bispecific binding molecules can be generated e.g., by fusing the hSIRPα-blocking Nbs with a tumor-opsonizing Nb and Fc moiety (see e.g., Hatterer et al., "Co-engaging CD47 and CD19 with a bispecific antibody abrogates B-cell receptor/CD19 association leading to impaired B-cell proliferation", MAbs (2019) 11: 322-334; Ma et al., "Preclinical development of a novel CD47 nanobody with less toxicity and enhanced anti-cancer therapeutic potential", J Nanobiotechnology (2020) 18:12) or CD40L expressed by activated T cells to bridge innate and adaptive immune responses (Lakhani et al., "Phase 1 dose escalation study of the agonist redirected checkpoint, SL-172154 (SIRPα-Fc-CD40L) in subjects with platinum-resistant ovarian cancer", Journal for ImmunoTherapy of Cancer (2021) 9: A459-A459). To address rapid renal clearance, which is a major drawback of the small-sized Nbs, other modifications such as PEGylation, addition of an albumin-binding moiety, or direct linkage to carrier proteins can be considered to extend their systemic half-life (t½) and efficacy (see, e.g., Griffiths et al., "Half-life extension and non-human primate pharmacokinetic safety studies of i-body AD-114 targeting human CXCR4", MAbs. Taylor & Francis (2019), pp. 1331-1340; Hanke et al., "A bispecific monomeric nanobody induces spike trimer dimers and neutralizes SARS-CoV-2 in vivo", Nature communications (2022) 13: 1-11).

Beside developing inhibitory hSIRPα Nbs, within the present invention, binders were identified to elucidate the presence and infiltration of the myeloid cell population within the TME and the primary and secondary lymphatic organs using PET-based non-invasive *in vivo* imaging. Current diagnostic methods are based on histology and thus require biopsies. However, invasive sampling and endpoint analyses can be associated with severe side effects and also limit the predictive value of such diagnostic approaches. Non-invasive *in vivo* whole-body molecular imaging techniques, in particular PET, which rely on target-specific tracer molecules, and as disclosed and claimed herein, represent a powerful method to monitor and quantify cell populations present within the TME and the lymphatic organs and thereby support individual therapy decisions.

Detailed epitope determination was performed by HDX-MS of non-hSIRPaD1-binding Nbs. As a result, hSIRPα-S36 Nb, a high-affinity binding candidate that does not block the hSIRPα/hCD47 interface or modulate ADCP was selected. For this candidate, efficient site-directed radiolabeling was achieved by replacing all four lysines with arginine within the hSIRPα Nb sequence. Importantly, this protein engineering approach did neither affect stability nor binding properties of the hSIRPα-S36 Nb . Compared to other, more elaborate, and less effective labeling strategies such as the sortagging, this resulted in rapid chelator conjugation by applying straightforward NCS chemistry.

⁶⁴Cu-hSIRPα-S36_{K>R} Nb-PET/MR imaging in tumor-bearing newly generated hSIRPa/hCD47 KI mice revealed rapid recruitment and sustained accumulation of our radiotracer in myeloid-enriched tumors and lymphatic organs with low background signal. Also, a significantly enhanced ⁶⁴Cu-hSIRPα-S36_{K>R} Nb uptake in MC38 adenocarcinomas of hSIRPa/hCD47 KI mice was observed, suggesting specific targeting of myeloid cells within the TME. Most importantly, an enhanced ⁶⁴Cu-hSIRPα-S36_{K>R} Nb uptake in tumors and lymphatic organs of murine SIRPα and CD47 expressing wt mice could not be detected. Beyond the crucial role of myeloid cells in tumor progression and cancer immunotherapy resistance, non-invasive *in vivo* monitoring of the biodistribution, density and dynamic changes of the myeloid cell compartment during inflammatory processes enables numerous innovative applications in infectious and autoimmune diseases. As examples, infiltration of lung macrophages during SARS-CoV-2 infection is a key driver of hyperinflammatory lung injury. Moreover, the occurrence of myeloid cells in diseased tissues is a hallmark of several autoimmune diseases such as systemic sclerosis, rheumatoid arthritis, and inflammatory bowel disease, as these cells heavily contribute to the disease initiation, development and progression.

Thus, the targeting SIRPα as a prominent marker for myeloid cells by means of the nanobodies of the invention represents a major benefit in the clinical setting especially in comparison to current clinically developed PET tracer molecules such as TSPO and ⁶⁸Ga anti-MMR Nb, which exclusively visualize subsets of the TAM population.

In addition, since the hSIRPα-S36 Nb detects both hSIRPα allele variants, applicability to the entire patient population is given. To minimize the radiation exposure to patients, Nbs can also be radiolabeled with ¹⁸F with a short t½ of only 110 min enabling imaging within the first few hours post tracer injection due to the fast clearance of the Nbs and most importantly repetitive immune cell tracer investigations and thus longitudinal studies.

In summary, the present invention demonstrates for the first time the generation and detailed characterization of hSIRPα-specific Nbs for potential therapeutic and diagnostic applications.

## Claims

1. A nanobody which binds to a member of the human SIRP family, wherein the nanobody binds at least to human SIRPα, wherein the nanobody does not have cross-reactivity with murine SIRPα, the nanobody comprising
a) the amino acid sequences (i) EFRLDEYA (SEQ ID NO: 1) as CDR1, (ii) ISRSGSNT (SEQ ID NO: 2) as CDR2 and (iii) AADLTSHYLSCSSYTDYNS (SEQ ID NO: 3) as CDR3; or
b) the amino acid sequences (i) GFSSSYYA (SEQ ID NO: 4) as CDR1, (ii) ITRSGDTT (SEQ ID NO: 5) as CDR2 and (iii) ALWAWAGSGRLRCTASEYGH (SEQ ID NO: 6) as CDR3; or
c) the amino acid sequences (i) GFTLDYYS (SEQ ID NO: 7) as CDR1, (ii) ITASGSST (SEQ ID NO: 8) as CDR2 and (iii) AAEPCTVVAGILQAPTSDFGS (SEQ ID NO: 9) as CDR3; or
d) the amino acid sequences (i) GFTLDYYA (SEQ ID NO: 10) as CDR1, (ii) ISSDGGGNT (SEQ ID NO: 11) as CDR2 and (iii) AAGPTYYSGSYCRDPHSDYDY (SEQ ID NO: 12) as CDR3; or
e) the amino acid sequences (i) GETLDYYA (SEQ ID NO: 13) as CDR1, (ii) ISSRGDNT (SEQ ID NO: 14) as CDR2 and (iii) VAIKDSWQNYYCSVGWVEYDV (SEQ ID NO: 15) as CDR3; or
f) the amino acid sequences (i) GFTFSSYD (SEQ ID NO: 16) as CDR1, (ii) IKSGGGRT (SEQ ID NO: 17) as CDR2 and (iii) GSLDNSGAYTG (SEQ ID NO: 18) as CDR3; or
g) the amino acid sequences (i) GFSFRDSA (SEQ ID NO: 19) as CDR1, (ii) IYSDGST (SEQ ID NO: 20) as CDR2 and (iii) APKGVVADEGDY (SEQ ID NO: 21) as CDR3;
h) the amino acid sequences (i) GRTFRAYA (SEQ ID NO: 32) as CDR1, (ii) IRWIGGTP (SEQ ID NO: 33) as CDR2 and (iii) AAEETQKGSGLGHDGRHYDY (SEQ ID NO: 34) as CDR3; or
i) amino acid sequences that have at least 85% sequence homology with the amino acid sequences as defined in a), b), c), d), e), f), g), or h); or
or a functionally conservative variant of the nanobody as defined in any of a), b), c), d), e), f), g), or h) comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 10, SEQ ID No. 11, and SEQ ID No. 12; or SEQ ID No. 13, SEQ ID No. 14, and SEQ ID No. 15; or SEQ ID No. 16, SEQ ID No. 17, and SEQ ID No. 18; or SEQ ID No. 19, SEQ ID No. 20, and SEQ ID No. 21; or SEQ ID NO: 32, SEQ ID NO: 33 and SEQ ID NO: 34.

2. The nanobody of claim 1, wherein the nanobody binds to i) domain D1, to ii) domain D2, or iii) to domain D3 of the extracellular region of human SIRPα.

3. The nanobody of claim 1 or 2, wherein the nanobody is an hSIRPα-CD47-interaction-blocking hSIRPα nanobody, a myeloid cell- or T cell-depleting nanobody, or an inert hSIRPα nanobody.

4. The nanobody of any of claims 1 to 3, comprising four framework regions (FR1 to FR4) and three complementarity determining regions (CDR1 to CDR3), the three complementarity determining regions consisting of
(i) one of the amino acid sequences a), b), c), d), e), f), g), or h), or of
(ii) an amino acid sequence that has at least 85% sequence homology with one of the amino acid sequences a), b), c), d), e), f), g), or h), or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9; or SEQ ID No. 10, SEQ ID No. 11, and SEQ ID No. 12; or SEQ ID No. 13, SEQ ID No. 14, and SEQ ID No. 15; or SEQ ID No. 16, SEQ ID No. 17, and SEQ ID No. 18; or SEQ ID No. 19, SEQ ID No. 20, and SEQ ID No. 21; SEQ ID No. 32, SEQ ID No. 33, and SEQ ID No. 34;
wherein preferably, the nanobody comprises or consists of an amino acid sequence selected from the group consisting of the amino acid sequences SEQ ID NO. 22, SEQ ID NO. 23, SEQ ID NO. 24, SEQ ID No. 25, SEQ ID No. 26, SEQ ID No. 27, SEQ ID No. 28, SEQ ID No. 35; or wherein the nanobody comprises or consists of an amino acid sequence that has at least 85% sequence homology with one of the amino acid sequences SEQ ID NO. 22, SEQ ID NO. 23, SEQ ID No. 24 or SEQ ID No. 25, SEQ ID No. 26, SEQ ID No. 27, SEQ ID No. 28, or SEQ ID No. 35.

5. The nanobody of any of claims 1 to 4, wherein in the nanobody amino acid sequences lysine residues are exchanged with arginine; wherein, preferably, the nanobody comprises or consists of an amino acid sequence selected from the group consisting of the amino acid sequences SEQ ID NO. 29, SEQ ID NO. 30 or SEQ ID NO. 31.

6. The nanobody of any of claims 1 to 5, wherein the nanobody is ,directly or indirectly, associated with at least one of a detectable label and/or a therapeutically or pharmacologically active agent.

7. The nanobody of claim 6, wherein the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuclides, or magnetic particles.

8. A nucleic acid comprising or consisting of a nucleic acid sequence coding for the nanobody as claimed in any of claims 1 to 6, optionally linked to another nucleic sequence.

9. The nanobody of any of claims 1 to 7, or the nucleic acid of claim 8, for use in diagnostic or prognostic methods, or for use as medicament, preferably for blocking the interaction of hSIRPα with CD47, or for target cell depletion, wherein the target cell preferably is a myeloid cell or T-cell.

10. The nanobody of any of claims 1 to 7 for use as contrast agent in non-invasive medical imaging *in vivo,* preferably for identifying the presence, absence, and/or amount of SIRPα⁺ cells.

11. The nanobody of any of claims 1 to 7 for use in the treatment of a solid tumor, cancer, leukemia, a lymphoma, an inflammatory disease, a neurological disorder, or a chronic infection, or an autoimmune disease.

12. The nanobody for use of claim 11, wherein the nanobody is used in combination with at least one therapeutically or pharmacologically active agent, wherein preferably the therapeutically or pharmacologically active agent is selected from the group consisting of an anticancer or antitumor agent, wherein further preferably the antitumor agent is a therapeutic antibody.

13. A multiparatopic, biparatopic, bivalent or trivalent construct comprising at least one nanobody as claimed in any of claims 1 to 7 or a combination thereof.

14. A method for determining and/or imaging and/or monitoring the distribution, presence and/or amount of hSIRPαa⁺ cells in a biological sample, the method comprising: contacting the biological sample or tissue with the nanobody as claimed in any of claims 1 to 7, and determining and/or imaging and/or monitoring the distribution, presence and/or amount of hSIRPα⁺ cells in the biological sample or tissue.

15. The method of claim 14, wherein the nanobody is detectably labeled, preferably radiolabeled, and wherein the in the determining and/or imaging and/or monitoring step the presence, or absence, or distribution, or amount of the label is detected.

16. A pharmaceutical composition comprising at least one of the nanobodies as claimed in any of claims 1 to 7, and/or at least one construct as claimed in claim 13, the pharmaceutical composition optionally further comprising: (i) a pharmaceutically acceptable carrier or excipient, and/or (ii) at least one therapeutically or pharmacologically active agent, wherein preferably the therapeutically or pharmacologically active agent is selected from the group consisting of an anticancer or antitumor agent, wherein further preferably the anti-tumor agent is a therapeutic antibody.

17. A cell line producing a nanobody of any of claims 1 to 7.

18. A kit comprising at least one nanobody of any of claims 1 to 7, the nucleic acid as claimed in claim 8, or a cell of a cell line of claim 17; and a detectable marker.
